# EUROPEAN PATENT SPECIFICATION

(11) **EP 1 047 436 B1**
(45) Date of publication and mention of the grant of the patent: **21.10.2009**
(21) Application number: 99901314.7
(22) Date of filing: 13.01.1999
(51) Int. Cl.: A61K 33/06, A61K 31/185, A61P 25/14

(54) **N-ACETYLHOMOTAURINATES FOR USE IN TREATING HYPERKINESIAS**
N-ACETYLHOMOTAURINATE ZUR VERWENDUNG IN DER BEHANDLUNG VON HYPERKINESIE
N-ACÉTYLHOMOTAURINATES POUR UTILISATION DANS LE TRAITEMENT DE LA HYPERKINÉSIE

(30) Priority: 13.01.1998 US 6641; 18.11.1998 US 193892; 04.01.1999 US 224829
(43) Date of publication of application: 02.11.2000
(73) Proprietor: Synchroneuron, LLC, Providence, RI 02906 (US)
(72) Inventor: FOGEL, Barry, S., Waban, Massachusetts 02468-0001 (US)
(74) Representative: Kirkham, Nicholas Andrew
(86) International application number: PCT/US1999/000144
(87) International publication number: WO 1999/036064

(56) References cited:
- US-A- 4 355 043
- US-A- 4 355 043
- US-A- 4 985 256
- US-A- 5 527 810
- US-A- 5 866 585
- GOZLAN H ET AL: "NMDA and GABAA receptors, NO and redox modulation. Fully oxidized NMDA receptors can still act as a source of NMDA receptor-driven plasticity" TRENDS IN PHARMACOLOGICAL SCIENCES, vol. 17, no. 5, 1 May 1996 (1996-05-01), page 187-189 XP004034550
- RASCOL O ET AL: "PHARMACOLOGIE CLINIQUE DES DYSKINESIES INDUITES PAR LA L-DOPA CHEZ LES MALADES PARKINSONIENS" THERAPIE, vol. 53, no. 1, 1 February 1998 (1998-02-01), pages 43-48, XP002094840 ISSN: 0040-5957
- S M PAPA ET AL: "LEVODOPA-INDUCED DYSKINESIA IMPROVED BY A GLUTAMATE ANTAGONIST IN PARKINSONIAN MONKEYS" ANNALS OF NEUROLOGY, vol. 39, 1 May 1996 (1996-05-01), pages 574-578, XP002090027 ISSN: 0364-5134
- RICHTER A ET AL.: "Antidystonic effects of the NMDA receptor antagonists memantine, MK-801 and CGP 37849 in a mutamnt hamster model of paroxysmal dystonia" NEUROSCI LETT, vol. 133, no. 1, 1991, page 57-60 XP002108849
- BERTON F ET AL.: "Acamprosate enhances N-methyl-D-aspartate receptor-mediated neurotransmission but inhibits presynaptic gabaB receptors in nucleus accumbens neurons" ALCOHOL.: CLIN.EXP.RES., vol. 22, no. 1, - 1998 pages 183-191, XP002108850
- BARTHOLINI G: "GABA receptor agonists: pharmacological spectrum and therapeutic actions" MED. RES. REV., vol. 5, no. 1, - 1895 pages 55-75, XP002108851
- CORSI M ET AL: "Co-agonism in drug-receptor interaction: illustrated by the NMDA receptors" TRENDS IN PHARMACOLOGICAL SCIENCES, vol. 17, no. 6, 1 June 1996 (1996-06-01), page 220-222 XP004034563 ISSN: 0165-6147

## Description

### Background of the Invention

This invention concerns the treatment of three major types of movement disorders 1) tardive dyskinesia (TD), tardive dystonia and related movement disorders, induced by exposure to neuroleptic (antipsychotic) drugs; 2) focal dystonias not related to medications, including blepharospasm, Meige syndrome, torticollis, spasmodic dysphonia, and writer's cramp; and 3) tics, including multiple tics and Gilles de la Tourette syndrome (TS).

Movement disorders affect a significant portion of the population, causing disability as well as distress. Tardive dyskinesia (TD) is a chronic disorder of the nervous system, characterized by involuntary, irregular rhythmic movements of the mouth, tongue, and facial muscles. The upper extremities also can be involved. These movements may be accompanied, to a variable extent, by other involuntary movements and movement disorders. These include rocking, writhing, or twisting movements of the trunk (tardive dystonia), forcible eye closure (tardive blepharospasm), an irresistible impulse to move continually (tardive akathisia), jerking movements of the neck (tardive spasmodic torticollis), and disrupted respiratory movements (respiratory dyskinesia). The vast majority of TD cases are caused by the prolonged use of antipsychotic drugs (neuroleptics). A relatively small number are caused by the use of other medications, such as metoclopramide, that, like neuroleptics, block dopamine receptors. TD often manifests or worsens in severity after neuroleptic drug therapy is discontinued. Resumption of neuroleptic therapy will temporarily suppress the involuntary movements, but may aggravate them in the long run.

TD is also associated with a variable degree of cognitive impairment. Cognitive dysfunction associated with TD may involve attention, concentration, memory, or executive functions such as judgment or abstract reasoning. (see, e.g., Sachdev et al., Acta Psychiatr Scand 93:451, 1996; Waddington & Youssef, Psychol. Med. 26:681, 1996; Swartz, Neuropsychobiology 32:115, 1995). The cognitive impairment associated with TD usually is seen as a marker of underlying differences in brain function that predispose the patient to TD. However, it may also be due to the TD itself, and may be either irreversible, or partially reversible if the TD is successfully treated.

Tardive dyskinesia (TD) affects approximately 15-20% of patients treated with neuroleptic drugs (Khot et al., Neuroleptics and Classic Tardive Dyskinesia, in Lang AE, Weiner WJ (eds.): Drug Induced Movement Disorders, Futura Publishing Co., 1992, p. 121- 166). Neuroleptics are used to treat several common psychiatric disorders, including schizophrenia and related psychoses (estimated prevalence 1%), mood disorders with psychotic features (estimated minimum prevalence 0.5%), and Alzheimer's disease with psychosis or agitation (estimated minimum prevalence at 0.5%). Assuming that half of those in need of neuroleptic treatment receive it, it follows that TD affects hundreds of thousands of people in the United States alone. The cumulative incidence of TD is substantially higher in women, in older people, and in those being treated with neuroleptics for conditions other than schizophrenia, such as bipolar disorder (manic-depressive illness) (see, e.g., Hayashi et al., Clin. Neuropharmacol. 19:390, 1996; Jeste et al., Arch. Gen. Psychiatry, 52:756, 1995). Unlike the acute motor side effects of neuroleptic drugs, TD does not respond in general to antiparkinson drugs (Decker et al., New Eng. J. Med., October 7, p. 861, 1971).

The focal dystonias are a group of movement disorders involving the intermittent sustained contraction of specific muscle groups, resulting in recurrent abnormal posturing of some part of the body. The most common is spasmodic torticollis, which involves twisting of the neck. Other examples are blepharospasm, which involves involuntary eye closure or excessively forceful blinking, and writer' s cramp, which involves contraction of the muscles of the hand. Another less common focal dystonia involves the laryngeal muscles (spasmodic dysphonia). Other relatively rare dystonias involve muscle groups specific to a particular occupation, such as playing the violin. The prevalence of focal dystonias in one US county was estimated as 287 per million (Monroe County Study); this suggests that at least 70,000 people are affected in the US alone. Blepharospasm alone affects more than 25,000 people (Source: US FDA Web Site; page on Orphan Drug Act).

Tics are estimated to affect 1% to 13% of boys and 1% to 11% of girls, the male-female ratio being less than 2 to 1. Approximately 5% of children between the ages of 7 and 11 years are affected with tic behavior (Leckman et al., Neuropsychiatry of the Bas.Gang, December, 20(4): 839-861, 1997). The estimated prevalence of multiple tics with vocalization, i.e. Tourette's syndrome, varies among different reports, ranging from 5 per 10,000 to 5 per 1,000. Tourette's syndrome is 3-4 times more common in boys than girls and 10 times more common in children and adolescents than in adults (Leckman et al., *supra;* Esper et al, Tenn. Med., January, 90:18-20, 1997).

A tic is an abrupt repetitive movement, gesture, or utterance that often mimics a normal type of behavior. Motor tics include movements such as eye blinking, head jerks or shoulder shrugs, but can vary to more complex purposive appearing behaviors such as facial expressions of emotion or meaningful gestures of the arms and head. In extreme cases, the movement can be obscene (copropraxia) or self injurious. Phonic or vocal tics range from throat clearing sounds to complex vocalizations and speech, sometimes with coprolalia (obscene speech) (Leckman et al., *supra).* Tics are irregular in time, though consistent regarding the muscle groups involved. Characteristically, they can be suppressed for a short time by voluntary effort.

Gilles de la Tourette syndrome (TS) is the most severe tic disorder. Patients with TS have multiple tics, including at least one vocal (phonic) tic. TS becomes apparent in early childhood with the presentation of simple motor tics, for example, eye blinking or head jerks. Initially, tics may come and go, but in time tics become persistent and severe and begin to have adverse effects on the child and the child's family. Phonic tics present, on average, 1 to 2 years after the onset of motor tics. By the age of 10, most children have developed an awareness of the premonitory urges that frequently precede a tic. Such premonitions may enable the individual to voluntarily suppress the tic, yet premonition unfortunately adds to the discomfort associated with having the disorder. By late adolescence/early adulthood tic disorders can improve significantly in certain individuals. However, adults who continue to suffer from tics often have particularly severe and debilitating symptoms. (Leckman et al., *supra).*

The pathophysiology of movement disorders, specifically TD, has not been established definitively. It is well known that blockade of dopamine receptors will lead to an increased number of dopamine receptors, and therefore to an increased sensitivity to dopamine of striatal neurons. (see e.g., Andrews, Can JPsych 39:576, 1994; Casey, in Psychopharmacology: The Fourth Generation of Progress, Raven Press, 1995). The first major hypothesis about the pathophysiology of TD was that TD was the result of this hypersensitivity of striatal neurons to dopamine. In support of the "dopamine supersensitivity" hypothesis, it is noted that dopamine agonists can aggravate the disorder (Bezchibnyk-Butler & Remington, Can J. Psych.. 39:74, 1994). However, the dopamine supersensitivity hypothesis is *not* compatible with the observation that TD and Parkinsonism (a dopamine deficiency state) often exist together in the same patient.

Other studies have suggested that irreversible cases of TD may be related to excitotoxic damage to the basal ganglia (Andreassen & Jorgensen, Pharmacol. Biochem. Behav., 49(2):309-312, 1994; Tsai et al.,: Am J Psych, September 155:9, 1207-13, 1998). An acquired deficiency of the inhibitory neurotransmitter GABA has also been implicated in the development of TD (Delfs et al., Experimental Neurol., 133:175-188, 1995).

A widely-studied animal model of TD, that of vacuous chewing movements (VCM), has also yielded evidence for a glutamate-based excitotoxic mechanism in the development of the disorder (Meshul et al; Psychopharmacology (Berl), 125:238-47, 1996 Jun.; Andreassen et al; Br J Pharmacol, 199:751-7. 1996 Oct.) When administered to rats with VCM, ethanol acutely decreases the animal's orofacial movements. This effect is prevented if the rats are pre-treated with a benzodiazepine inverse agonist, suggesting that it is mediated by stimulation of GABA-A receptors by ethanol (Stoessl, Pharmacol. Biochem. Behav. July, 54:541-6, 1996 Jul.) Stoessl suggests that "GABAergic stimulation" deserves further investigation in the treatment of TD. He does not, however, advance the idea of treating TD with NMDA antagonists, nor suggest using memantine as a treatment for TD.

The physical manifestations of TD can resemble movement disorders associated with degenerative diseases such as Huntington's disease and Parkinson's disease. Patients with TD can show chorea (quick, irregular movements of the extremities) indistinguishable from that seen in cases of Huntington's disease. Neck, trunk and limb movements of TD can be indistinguishable from those of the peak-dose dyskinesia associated with prolonged treatment of Parkinsons disease with levodopa. The physical manifestations of tardive dystonia are nearly identical to the manifestations of the idiopathic dystonias, i.e., those not related to exposure to dopamine antagonists. (See the further discussion below.)

It is evident that similar mechanisms may be involved in the pathophysiology of tardive movement disorders and idiopathic focal dystonias. Positron emission tomography has shown that one specific dystonia, torticollis, is associated with neuronal hypermetabolism in the basal ganglia. It has been hypothesized that hyperactivity of a motor control loop involving the cerebral cortex, basal ganglia, and thalamus is responsible for the abnormal postures and movements (i.e. movements into and out of abnormal postures) characteristic of dystonia (Galardi et al., Acta. Neurol Scand, September, 94:172-6, 1996). Other studies have shown abnormal dopaminergic transmission or receptor function in patients with dystonia (see, e.g. Perlmutter et al., J Neurosci, January 15, 17:843-50, 1997). Of note, both too much or too little dopamine may be associated with dystonia, since patients with Parkinson's disease and dystonia can have the problem both at peak and trough levels of levodopa (Hallett, Arch. Neurol. May, 55:601-3, 1998). Although memantine has dopamine agonist activity, memantine has not been suggested as a treatment for focal dystonias.

The pathophysiology of tic disorders like, that of TD, has not yet been established definitively, although several plausible hypotheses have been set forth. The pathophysiology of tic disorder resembles the mechanism that may be involved in the pathophysiology of TD and focal dystonias. Excessive activity of a cortical-striatal-pallidal-thalamic-cortical sensorimotor loop has been implicated in the lack of motor impulse control associated with tic disorders (Zambian et al., Am. J. Psychiatry, Vol 154, September, 1997; Leckman et al., *supra*)*.* This hyperactivity may reflect excessive dopaminergic activity in the striatum, or a relative deficiency of inhibitory transmission. While dysfunction of the basal ganglia or their connections is likely to be present, the basal ganglia, thalamus, and motor cortex are anatomically normal in most cases.

### Treatment of TD

Recent research suggests that Vitamin E can reduce symptoms of TD modestly (Lohr & Caliguiri, J Clin Psychiatry 57; 167, 1996; Dabiri et al. Am. J. Psychiatry, June, 151(6):925-926, 1994). GABA agonists such as baclofen and various benzodiazepines have also been the subject of some positive reports and are widely used in practice to ameliorate the symptoms of TD, probably because their low toxicity justifies their use despite their limited efficacy. (Gardos & Cole, Psychopharmacology: The Fourth Generation of Progress, eds. Bloom and Kupfer, pp. 1503-1510, 1995). This review cited reports of variable benefits associated with other agents including propranolol, clonidine, cholinergic agonists, buspirone and calcium-channel antagonists. However, none of these has become a generally accepted treatment for either the movement or cognitive disorders associated with TD. (The author has had some success in treating TD with nimodipine, a calcium-channel antagonist with particularly good penetration of the CNS.)

In U.S. Patent Number 5,602,150, by Lidsky et al., it was proposed that the emergence of TD in patients receiving neuroleptics might be prevented by simultaneously administering taurine or taurine derivatives. Lidsky based his invention on the theory that TD is due to excitotoxic damage, and that taurine and taurine derivatives would protect patients against this damage. The recommendation of taurine is based on studies in a single animal model. The experiments reported do not deal with any therapeutic effects of taurine on established movements, either in the presence of continued neuroleptic administration or otherwise. Neither the patent nor the experiments cited in it predict or imply that taurine or derivatives will be beneficial for established movement disorders. Moreover, the mechanism proposed by Lidsky et al., *(supra)* is based on long-term neuroprotection. He neither infers, asserts, nor suggests that taurine or taurine derivatives might have any immediate, short-term effect on movement disorders.

Memantine is a drug approved in Europe for treatment of Parkinson's disease. Memantine, a congener of amantadine, is a N-methyl-D-aspartate type glutamate receptor blocker ("NMDA receptor antagonist" or "NMDA receptor blocker") as well as a dopamine agonist. Although memantine has been reported to alleviate some of the dyskinetic movements that can be seen in treated Parkinson' s disease, there are no reports of its use in humans to treat tardive dyskinesia, and at least one widely-reputed expert in the field of TD expressed surprise that any anti-Parkinson' s drug would be an effective agent against TD (Dilip Jeste, M.D., personal communication, 1997).

In U.S. Patent 4,122,193, it is reported that 1,3,5-trisubstituted adamantane, including 1-amino-3,5-dimethyl-adamantane is useful in the treatment of hyperkinesis in rats. The agent is also recommended as a treatment generally for hyperkinesis, in the context of Parkinson's disease, head tremors, thalamic tension conditions, and spastic conditions, and for "the activation of patients with akinetic cerebroorganic conditions". It is notable that, unlike TD, none of the underlying conditions described in that patent as the context of the hyperkinesis to be treated, is thought to be aggravated by dopamine agonists. Moreover, there is no recognition in the reference that 1-amino-3,5-dimethyl-adamantane acts as a NMDA-receptor blocker. Instead, the disclosure indicates that 1,3,5-trisubstituted adamantane compounds influence catecholamine metabolism, for instance by freeing dopamine or stimulating the receptors. This latter aspect suggests that the authors did not recognize that memantine could be an effective treatment of TD, for which administration of dopamine agonists in general goes against expert opinion.

In co-pending, commonly-owned applications Serial Nos., 08/861.801 and 09/006,641, incorporated herein by reference, treatments with memantine (a congener of amantadine and a N-methyl-D-aspartate type (NMDA) receptor blocker as well as a dopamine agonist), and acamprosate (a calcium salt of a derivative of the amino acid taurine and an indirect NMDA antagonist and GABA-A agonist), were advanced as effective treatments for both the movement and the cognitive disorders associated with TD, and were reported to be dramatically effective in several severely affected individuals. The cases described in those patent applications contain the first reports of the use of memantine for the treatment of TD.

### Treatment of focal dystonia

As noted above, a focal dystonia is a movement disorder involving recurrent abnormal posturing of some part of the body. The spasms of focal dystonia can last many seconds at a time, causing major disruption of the function of the affected area. Some of the focal dystonias are precipitated by repetitive movements; writers cramp is the best known example. Focal dystonia can involve the face (e.g., blepharospasm, mandibular dystonia), the neck (torticollis), the limbs (e.g., writer's cramp), or the trunk. Dystonia can occur spontaneously or can be precipitated by exposure to neuroleptic drugs and other dopamine receptor blockers (tardive dystonia). No systemic drug therapy is generally effective, but some drugs give partial relief to some patients. Those most often prescribed are anticholinergics, baclofen, benzodiazepines, and dopamine agonists and antagonists. The most consistently effective treatment is the injection of botulinum toxin into affected muscles.

The various focal dystonias tend to respond to the same drugs (i.e., treatments that are helpful for one focal dystonia generally have been helpful for others.) (Chen, Clin. Orthop, June, 102-6, 1998: Esper et al; Tenn. Med, January, 90:18-20, 1997; De Mattos et al., 4rq. Neuropsychiatry, March 54:30-6, 1996). Published clinical experience to date suggests that a new treatment that reduces the involuntary movements of one focal dystonia would be likely to do the same for the involuntary movements of another. Furthermore, the common symptoms, signs, and responses to medication of spontaneous (idiopathic) dystonia and neuroleptic-induced dystonia suggest that an effective treatment for a drug-induced focal dystonia will be effective for the same dystonia occurring spontaneously.

Blepharospasm, one of the focal dystonias, is a condition that involves continually recurring involuntary eye closure or excessive forceful blinking. Blepharospasm is one of the most common disorders of oculomotor function. It is variably regarded as a facial dyskinesia or a facial dystonia. When it occurs together with dystonia of the oral and mandibular regions, with or without involvement of the neck, it is referred to as Meige syndrome. Blepharospasm can significantly impair visual function. Patients can become unable to read, to drive an automobile, or to do any skilled work requiring visual control. Blepharospasm can occur spontaneously (idiopathic blepharospasm) and with a prevalence that increases with increasing age; most cases arise in the fifth and sixth decades of life (Holds et al., Am. Fam. Physician, June, 43:2113-20, 1991). It also can occur as a sequel to neuroleptic drug treatment (Ananth et al., Am. J. Psychiatry, April, 145:513-5, 1988; Kurata et al., Jpn. J Psychiatry. Neurol., December, 43:627-31, 1989; Sachdev et al., Med. J. Aust., March 20, 150:341-3, 1989) and perhaps treatment with other classes of psychotropic drugs (Mauriello et al., J Neuropathol, June, 18:153-7, 1998), either alone or in conjunction with tardive dyskinesia or other forms of tardive dystonia. Another report of 19 patients with severe tardive dyskinesia, stated that frequent eye blinking was the most frequent prodromal sign of the disorder (Gardos et al., *supra,* 1988). The oculomotor phenomena of idiopathic blepharospasm and Meige syndrome are identical with those seen in cases induced by neuroleptic treatment. Differences between idiopathic blepharospasm and tardive blepharospasm do not involve the ocular movements themselves. (Observed differences have involved family history and the likelihood that other non-ocular involuntary movements will be present.)

Though many substances have been tested for their ability to relieve blepharospasm, injection of botulinum toxin into orbicularis oculi muscles is the mainstay of treatment (Mauriello et al.. Br. J. Ophthalmol, December, 80:1073-6, 1996). These injections weaken the muscles responsible for eye closure, thereby mitigating the involuntary movements of those muscles. They may also indirectly influence oculomotor control by the central nervous system, by altering the input from motor nerve afferents. Botulinum toxin injections have become treatment of choice because they ameliorate symptoms in approximately 80% of patients - a much greater proportion than benefit from the numerous systemic drug treatments tried to date.

Movements associated with blepharospasm do not respond well to the systemic drug treatments employed to date. In one large case series, only 22% of blepharospasm patients treated with systemic medications got marked and persistent relief (Jankovic et al., Mov. Disord., May, 9:347- 349,1983). In another report, of the 13 patients with blepharospasm who did not do well with botulinum toxin injections, only 2 showed any improvement when given systemic drug therapy (Mauriello et al., Clin. Neurol. Neurosurg., August, 98:213-6, 1996)). Even botulinum toxin injections are not always efficacious. Surgery is sometimes recommended for patients who do not get relief from botulinum toxin injections (Elston et al., J. Neurol, January, 239:5-8, 1992).

Of the numerous systemic treatments tried for the treatment of blepharospasm, (see, for example, Arthurs et al., Can. J. Ophthalmol; February, 22:24-8, 1987; Casey et al., Neurology, July, 30:690-5, 1980, Jacoby et al., Invest. Ophthalmol. Vis. Sci., March, 31:569-76, 1990; Michaeli et al., Clin. Neuropharmacol., June, 11:241-9, 1988; Ransmayr et al., Clin. Neuropharmacol., February, 11:68-76, 1988) clonazepam, a GABA agonist, was the only drug consistently found useful (Jankovic et al., Ann. Neurol., April, 13:402-11, 1983). A combination of two GABA agonist agents, valproate and baclofen, was efficacious in a single case (Sandyk, et al., S Afr Med J, December, 64:955-6, 1983). Tetrabenazine, a dopamine depleting agent, alleviated involuntary movements in 4 of 6 patients with Meige syndrome, but the patients had many undesirable side effects including drowsiness, drooling and Parkinsonism (Jankovic, et al., Ann Neurol, January, 11:41-7, 1982). Because of such unpleasant side effects, tetrabenazine has not become a widely-used treatment for blepharospasm, tics or even tardive dyskinesia, despite the absence of other generally effective treatments for these conditions. Neuroleptics sometimes relieve symptoms of blepharospasm, but they do so less well than tetrabenazine, and patients treated with them run the risk of developing TD or other tardive movement disorders. In sum, though drugs that reduce dopaminergic transmission and GABA agonists have been employed with some benefit in the treatment of idiopathic blepharospasm, these types of medications have proved to be generally satisfactory treatments.

### Treatment of tics and Tourette's syndrome

Patients with moderate to severe motor and vocal tics are likely to require drug therapy. Many classes of neurological and psychiatric medications have been tried, but only neuroleptics, alpha-2 adrenergic agonists, and clonazepam have attained the status of standard treatments. (For recent reviews see Chappell et al., Neur. Clin. of North Am., 15(2), May 1997; Kurlan, Neurol. Clin., May, 15:403-409, 1997; Lichter et al., J. Child Neur., 11(2), March, 1996; Leckman et al., *supra;* Esper et al, Tenn. Med., January, 90:18-20, 1997; Scahill et al., J. Child Adolesc Phychopharmacol, 7(2), 1997; incorporated herein by reference). Unfortunately, all three of the commonly-used treatment for TS have significant drawbacks.

The most common therapies used for the treatment of tic disorders are the neuroleptics (i.e. dopamine antagonist antipsychotic drugs). Within this category, haloperidol and pimozide are most often used in the United States. Neuroleptic treatment usually will suppress the involuntary movements of tic disorders, with up to 85% of patients experiencing relief (Esper et al., *supra*)*.* The side effects of neuroleptic drugs include sedation, depression, parkinsonism, cognitive impairment, and tardive dyskinesia. Other tardive movement disorders can develop with prolonged use. The intolerability of side effects often leads patients to discontinue neuroleptic therapy for TS, while the risk of TD makes most physicians unwilling to use them in milder cases. Those with more severe TS must often make an unpleasant choice between distressing symptoms and distressing side effects. People with simple tics may experience emotional distress, embarrassment, impaired self-esteem, or physical injury if their tics are sufficiently violent. Yet, they usually will not be treated with neuroleptics because their side effects and long-term toxicity that are not acceptable in the treatment of relatively mild cases.

Other drug treatments for TS do not carry the risk of TD. But they are less efficacious than neuroleptics. The most common non-neuroleptic alternatives are alpha-2 adrenergic agonists such as clonidine. Unfortunately, fewer than 50% (perhaps as few as 25%) of patients treated with clonidine show clinically significant improvement of tic-related symptoms (Esper et al., *supra;* Chappell et al., *supra).* Further, many patients whose tics do respond to clonidine will have side effects that limit its use, most often hypotension or sedation.

Another non-neuroleptic treatment, clonazepam, a benzodiazepine with GABA-A and serotonergic actions, has some efficacy in the treatment of Tourette's syndrome (Steingard et al., J. Am Acad Child Adolesc Psychiatry, March-April, 33:394-9, 1994). Sedation and ataxia limit the dosage of clonazepam; the tolerable dose often is below that needed to suppress the patient's tics.

A new class of compounds that act as antagonists of brain serotonergic 5-HT₂ receptors initially showed promising results, although children and adolescents experience increase in sensitivity to side effects (Chappell et al., *supra*)*.* Additional alternatives that have received recent attention include antioxidant treatment (Rotrosen et al., Prost. Leuk. and Ess. Fatty Acids, 55(1 & 2), 1996), transcranial magnetic stimulation (Ziemann et al., *supra),* nicotine treatment (Sanberg et al., Pharmacol. Ther., 74(1)., 1997; Silver et al., J. Am. Acad. Adolesc. Psychiatry, Vol 35, December, 1996) and botulinum toxin treatment (Esper et al., *supra*)*.* While each of these treatments has offered clinically significant relief to individual patients, none has replaced neuroleptics as the treatment of choice. Clearly, there is a need for additional treatments for tics and TS that do not carry the side effects and long term risks of neuroleptics.

It has been suggested, on theoretical grounds, that future therapies for Tourette's syndrome might include glutamate antagonists, although a recent article proposing their use makes no mention of any specific drugs that might fulfill this role (Chappell et al., Neurol. Clin. May. 15(2):429-450, 1997).

### Magnesium and movement disorders

There is considerable evidence for abnormalities of magnesium status in patients with severe mental illness (see for example, Athanassenas et al., J. Clin. Psychopharmacol. August, 3:212-6, 1983; Alexander et al., Br. J. Psychiatry, August, 133:143-9, 1978; Kirov et al., Neuropsychobiology, 30(2-3):73-78, 1994; Wang et al, 1997; Yassa et al., Int. Pharmacopsychiatry, 14(1):57-64, 1979). Alexander et al. (*supra,* 1978) found that those schizophrenic patients developing extrapyramidal side effects from neuroleptics had, on average, lower magnesium levels than those not having such side effects. Neuromuscular excitability and anxiety are common acute manifestations of magnesium depletion. And, there are theoretical reasons to speculate that magnesium deficiency may contribute to a wide range of neurodegenerative disorders (Durlach et al. 1997, *supra*)*.*

Because magnesium deficiency can cause neuromuscular excitability (Durlach et al, Magnes Res, June, 10:169-95, 1997), it could potentially cause or aggravate movement disorders. Ploceniak, (Communications Libres, 91, suppII, 1990) reported, without details, that he had found magnesium supplementation useful in patients with bruxism (teeth grinding) and facial tics associated with tetany (susceptibility to muscle cramps typical of hypocalcemia). He did not, however, suggest that magnesium supplementation would help patients with Tourette's syndrome, or those with tics not due to magnesium deficiency. There has been no suggestion that magnesium deficiency is a cause of tardive dyskinesia, other tardive movement disorders, blepharospasm, or other focal dystonias, or that magnesium supplementation could be used to successfully treat or prevent movement disorders, in the absence of overt magnesium deficiency manifested by tetany.

Although the present day pharmacopeia offers a variety of agents to treat the movement disorders described above, none of these agents can prevent or cure these conditions. Furthermore, the most effective systemic drug treatments often are associated with intolerable side effects. Injections of botulinum toxin can be uncomfortable, must be repeated frequently, and often lose their efficacy over time. In addition, when used to treat dystonia of an upper extremity, they may weaken muscles needed for optimal function of the hands. There remains a clear cut need for new systemic treatments for TD, other tardive movement disorders, blepharospasm, and other focal dystonias, that have greater efficacy and fewer side effects than those currently available.

### Summary of the Invention

Essential and optional features of the present invention are set out in the accompanying main- and sub-claims respectively. Thus, the present invention provides agents and preparations for treating movement disorders including TD, other tardive movement disorders, tic disorders, blepharospasm, and other focal dystonias, in humans. The present invention also provides agents and preparations for reducing involuntary movements characteristic of patients with movement disorders

The invention provides, for use in a method for reducing involuntary movements characteristic of patients with hyperkinetic or dyskinetic movement disorders, a pharmacological agent, that both (i) acts directly or indirectly as an agonist at GABA-A receptors and (ii) decreases NMDA-type glutamate neurotransmission by an indirect or modulatory mechanism. Specific instances include calcium N-acetylhomotaurinate (acamprosate), magnesium N-acetylhomotaurinate, other salts of N-acetylhomotaurinate, derivatives of N-acetylhomotaurinate with similar pharmacodynamic effects on GABA and NMDA-type glutamate neurotransmission, and pro-drugs that are metabolized in the liver, blood, or brain to yield N-acetylhomotaurinate or a derivative with similar pharmacodynamic effects.

The present disclosure also provides a method for treating movement disorders by combining magnesium or a non-competitive NMDA receptor antagonist with memantine or another compound or mixture thereof (specifically including those enumerated in the previous paragraphs) that decreases the postsynaptic response to glutamate at NMDA-type receptors. For example, dextromethorphan can be combined with memantine and magnesium for the treatment of movement disorders. There is also disclosed, a combination of magnesium or a non-competitive NMDA receptor antagonist with memantine or another compound or mixture thereof that simultaneously decreases the postsynaptic response to glutamate at NMDA-type receptors and also directly or indirectly increases GABA-A transmission. In preferred embodiments, magnesium is used as a non-competitive NMDA receptor antagonist. (Memantine functions as an NMDA receptor antagonist via blockade of calcium ion channels.)

Magnesium can augment the effect of pharmacological agents used to treat movement disorders including tics and TD, and, by extension, TS and blepharospasm and other focal dystonias, whether caused by neuroleptic exposure or not. Synergistic activity is shown between magnesium and other pharmacological agents that act as NMDA receptor antagonists and also agents that act as NMDA receptor antagonists and simultaneously as enhancers of GABA-A transmission. In one embodiment, any combination of agents that act as NMDA receptor antagonists, with or without magnesium, are used for treatment of movement disorders. Alternatively, it is disclosed that magnesium alone is used to reduce symptoms associated with movement disorders.

It is further disclosed that supplementation with magnesium is used to prevent movement disorders in people already at risk for them, by reducing the risk, or by delaying the onset of the movement disorder for which they are at risk. In particular, it is asserted that magnesium deficiency is a risk factor for the development of TD in patients receiving neuroleptics, and that magnesium supplementation may prevent the development of TD, particularly in patients prone to magnesium deficiency, including elderly women, alcoholics, diabetics, people taking diuretics, and malnourished individuals.

In other embodiments, any combination of agents that act as NMDA receptor antagonists together with one or more agents that facilitate GABA-A neurotransmission (by acting as GABA-A receptor agonists, by increasing GABA-A release, or by increasing the post-synaptic response to GABA-A receptor stimulation), with or without magnesium, are used for treatment of movement disorders.

A pill combining an one or more agents that act as NMDA-type glutamate receptor antagonists and magnesium with or without an agent that acts as a GABA agonist is proposed as a specific vehicle for the delivery of this combined therapy. In addition, other oral preparations are suggested, the mixture can be delivered in a syrup, elixir, or time release capsule. The latter is suggested as a method for prolonging the duration of action of a dose of the mixture.

### Definitions

"Tardive dyskinesia": As used herein "tardive dyskinesia" is meant to include tardive dystonia and other movement disorders related to long-term neuroleptic use. The abbreviation TD may be used in place of the term "tardive dyskinesia". Also, the set of conditions comprised by TD in this application can also be referred to as "tardive dyskinesia and related tardive movement disorders".

"Blepharospasm": As used herein, "blepharospasm" includes Meige syndrome, which is a combination of blepharospasm and dystonia of the face and/or neck.

"Focal dystonia": As used herein, "focal dystonia" includes blepharospasm and Meige syndrome, spasmodic torticollis, spastic dysphonia, writer's cramp, musician's cramp and other occupational dystonias.

"Tourette's syndrome": "Tourette's syndrome" as used herein is synonymous with "Gilles de la Tourette syndromes", "Tourette syndrome", "Tourette disorder", and similar expressions. The abbreviation TS may be used in place of any of these terms.

"NMDA receptor antagonist": As used herein, "NMDA receptor antagonist" is any molecule that inhibits or diminishes the post-synaptic response of NMDA-type glutamate receptors to glutamate.

"NMDA-type glutamate neurotransmission": "NMDA-type glutamate neurotransmission" is used herein broadly, to refer to anything that would decrease NMDA-glutamate transmission, whether it acts before the synapse, at the glutamate receptor binding site, at a modulatory site such as the glycine modulatory site, within the ion channel, within the cell membrane, or inside the neuron. This also includes any substance that reduces release of glutamate at synapses with NMDA receptors, alters the binding of glutamate to NMDA receptors or alters the number or type of NMDA receptors.

"Acamprosate": As used herein, "acamprosate" refers to calcium N-acetylhomotaurinate. These two terms may be used interchangeably. "N-acetylhomotaurinate" and "acetylhomotaurinate" are also used interchangeably.

"Acamprosate and related compounds": "Acamprosate and related compounds" refers to calcium acetylhomotaurinate, magnesium acetyllhomotaurinate, other salts of N-acetylhomotaurinate, acetylhomotaurine base, homotaurine base and homotaurine salts, derivatives of homotaurine or acetylhomotaurine that have similar pharmacodynamic activity with respect to GABA-A and NMDA-type glutamate transmission, and pro-drugs that are metabolized in the blood, liver, or brain to yield acetylhomotaurinate or derivatives with similar pharmacodynamic activity with respect to GABA-A and NMDA-type glutamate transmission. Acamprosate decreases the intra cellular response of neurons stimulated by glutamate at the NMDA receptor, and enhances GABA-A transmission, at least in part by an antagonist effect on pre-synaptic GABA-B inhibitory autoreceptors. For ease of expression, I refer to acamprosate and similar compounds as: "GABA agonists and NMDA antagonists", "GABA-A agonists and NMDA-antagonists", "agents that increase GABA transmission and decrease NMDA-type glutamate transmission", "GABA agonists and glutamate antagonists", and "up regulators of GABA transmission and down-regulators of NMDA-type glutamate transmission".

"GABA-A transmission": "GABA-A transmission refers to the pharmacodynamic phenomena associated with the activation of GABA-A receptors by GABA. Enhancement of GABA-A transmission may involve increasing the release of GABA, decreasing its metabolism, increasing receptor binding, or increasing the cellular effects of receptor binding

"GABA-A receptor agonist": "GABA-A receptor agonist", as used herein refers to molecules that are capable of binding to active or modulatory sites on the GABA-A receptor to enhance GABA-A transmission. (as defined above)

"Effective": "Effective" as used herein in reference to dosage of a medication, refers to the administration of a specific amount of a pharmacologically active agent tailored to each individual patient manifesting symptoms of a particular movement disorder (e.g. TD, other tardive movement disorders, tic disorders, blepharospasm, and other focal dystonias) sufficient to cause a reduction or improvement in the patient's involuntary movements or any of the other symptoms associated with the movement disorder, with tolerable adverse effects. Symptoms of movement disorders, as referred to herein, refer not only to involuntary movements, but also to any and all impairments of physical, instrumental, social, and occupational functioning, including visual function, cognitive function, and use of the hands, that are attributable to the involuntary movements, or to the brain dysfunction that underlies them.

Experimentally, doses of memantine ranging from 10 mg to 30 mg have been shown to be effective, as have doses of dextromethorphan ranging from 30 mg four times a day to 60 mg four times a day. Doses of acamprosate ranging from 333 mg to 666 mg administered three to four times daily are effective. A person skilled in the art will recognize that the optimal dose of a pharmaceutical agent to be administered will vary from one individual to another. Dosage in individual patients should take into account the patients height, weight, rate of absorption and metabolism of the medication in question, the stage of the disorder to be treated, and what other pharmacological agents are administered concurrently.

"Movement disorder": "Movement disorder", as used herein, is used to refer to all forms of abnormal and involuntary movements. Movement disorders include, for example, tardive dyskinesia (TD), tics, Gilles de la Tourette syndrome (TS), Parkinsons disease, Huntingtons disease, and focal dystonias such as blepharospasm. Specific movement disorders that are the subject of this application include, without limitation, TD, other tardive movement disorders, blepharospasm and other focal dystonias, whether or not the latter are associated with exposure to neuroleptic drugs or other dopamine antagonists:

"Tic disorder": "Tic disorder" as used herein, refers to an abrupt repetitive movement, gesture, or utterance that often mimics a fragment of purposeful behavior. Tics are characterized by stereotyped, repetitive, but irregularly rhythmic involuntary movements. They include both motor tics and vocal (phonic) tics. Tic disorders include, for example, simple tics, multiple tics and Gilles de 1a Tourette syndrome, defined as multiple tics with vocalizations.

### Detailed Description of the Invention

The present disclosure relates to the treatment of movement disorders including, without limitation, TD, other tardive movement disorders, tics, Tourettes, blepharospasm and other focal dystonias, whether or not the latter are associated with exposure to neuroleptic drugs or other dopamine antagonists. These movement disorders likely involve some of the same physiological mechanisms and therefore will likely be responsive to the same treatments. I have discovered that memantine, a drug used in the treatment of Parkinson's disease, but not contemplated for use in treatment of tardive dyskinesia, tardive dystonia, or focal dystonia whether or not related to neuroleptics or other drugs, is effective in reducing the involuntary movements, cognitive symptoms, and functional impairment associated with tardive dyskinesia. I have also discovered that memantine is an effective treatment of tics and related tic disorders. In another aspect of the present invention, I have discovered that an agent used in the treatment of abstinent alcoholics, not contemplated for use in treatment of tardive dyskinesia or other movement disorders, including Tourette's syndrome and tics, is effective in reducing the hyperkinesia and dyskinesia of patients with movement disorders.

Several years ago, I hypothesized that TD represents a form of non-linear oscillation in neural circuits involving the basal ganglia, and that oscillation might be reduced by agents that block excitatory neurotransmission. PET scan studies have demonstrated increased metabolism in the globus pallidus and primary motor cortex in schizophrenic patients with TD, but not in those without TD (Pahl et al., J. Neuropsych Clin Neurosci 7:457, 1995). This suggests that TD is associated with hyperactivity in a motor control circuit, which might be part of the putative nonlinear oscillator.

As noted above, I have advanced the hypothesis that agents which act to reduce the gain in a motor control circuit through the striatum, may have a beneficial action on TD and related movement disorders including tardive movement disorders, focal dystonias, tics and Tourette's. For example, GABA is an inhibitory neurotransmitter in the striatum. Thus, support for my hypothesis comes from animal evidence indicating that agents that directly or indirectly stimulate GABA receptors can decrease neuroleptic-induced dyskinesias (Gao et al. J Neural Transmission 95:63, 1993; Stoessl, Pharmacol. Biochem. Behav., 54:541, 1996). Rats with neuroleptic-induced dyskinesia demonstrate decreased striatal levels of glutamic acid decarboxylase, the rate-limiting enzyme in the production of GABA (Delfs et al., Exp. Neurol., 133:175, 1995).

Without limiting the biochemical mechanism to that described here, it appears that drugs that act to reduce the gain in the hypothesized oscillator circuit would reduce the involuntary movements of tardive dyskinesia. GABA, glutamate, and dopamine are the principal neurotransmitters in the circuit. Other neurotransmitters, including norepinephrine, serotonin, acetylcholine and endogenous opiates are hypothesized to have indirect actions on the oscillator circuit. In my co-pending patent application, Serial No. 08/861,8.01, and the teachings of which are incorporated herein by reference, I disclosed that certain antagonists (and agonists) of excitatory neurotransmitters are effective in treating both the movement and cognitive disorders associated with TD, tardive dystonia, tics, and movement disorders that share a similar biochemical mechanism.

### Acamprosate

I disclose that acamprosate, a GABA-receptor agonist that also diminishes the postsynaptic response of NMDA-type receptors to glutamate can ameliorate TD as well as related involuntary movements and cognitive symptoms. For example, according the theory presented herein, a GABA agonist with concurrent effects on glutamate transmission reduces the severity of the involuntary movements associated with TD. Such a GABA agonist alleviates focal dystonias, for example blepharospasm associated with TD and by extension idiopathic blepharospasm, which is likely to share a common mechanism, in light of the response of both to dopamine antagonists, to GABA agonists, and to botulinum toxin injections. To further this point, an expert on blepharospasm, Dr. Gary Borodic of the Harvard Medical School, states that neuroleptic-induced (tardive) blepharospasm is in general less responsive to medications than the spontaneous kind (Borodic, personal communication, 1998). If this is so, a treatment effective for tardive blepharospasm is especially likely to be helpful for spontaneous blepharospasm.

Likewise, treatment with acamprosate will likely ameliorate symptoms associated with Meige syndrome, which is blepharospasm accompanied by dystonic movements of the neck and lower face. Also disclosed in the present application is that acamprosate dramatically diminishes dyskinetic movements associated with tic disorders, including both single and multiple tics. Furthermore, I propose that acamprosate and other agents that both (i) decrease NMDA-type glutamate neurotransmission, and (ii) increase GABA-A receptor neurotransmission are useful in the treatment of a common and severe type of tic disorder, Tourette's syndrome, which is characterized by multiple motor and phonic tics.

Acamprosate (calcium N-acetylhomotaurinate) is the calcium salt of homotaurine, a derivative of the amino acid taurine. It is used clinically in the treatment of abstinent alcoholics to reduce or inhibit their craving for alcohol. Acamprosate, which is chemically similar to the inhibitory neurotransmitter GABA, is a GABA agonist, particularly at GABA-A receptors. Moreover, it reduces the postsynaptic response of NMDA-type glutamate receptors and reduces calcium influxes through voltage-operated channels. (Wilde & Wagstaff, Drugs, 53:1039-53, 1997)

Acamprosate is a particularly attractive drug for treating chronic movement disorders, because of its very low toxicity. In controlled trials for alcoholism treatment involving 3,338 patients, acamprosate had no severe medical or neurological side effects. Indeed, the rate of subject dropout was identical in the group receiving acamprosate treatment and in the group receiving a placebo (Wilde and Wagstaff, Drugs, June, 53(6):1038-53, 1996). This is in stark contrast to existing systemic treatments for TD and TS. For these, as noted above, intolerable side effects are common, and impose a major limitation on their clinical utility.

The above hypothesis regarding a motor control circuit involving GABA (via GABA-A receptors) and glutamate (via NMDA receptors) implies that any drug that is a GABA agonist and an NMDA-type glutamate antagonist can ameliorate dyskinetic movements. Acamprosate (calcium N-acetylhomotaurinate) is a specific example of such a drug for which I offer direct evidence in humans of efficacy in the treatment of dyskinesia. Other examples of such drugs include other salts of N-acetylhomotaurine, derivatives of taurine and homotaurine with similar effects on GABA and NMDA-type glutamate transmission, and pro-drugs that are metabolized in the liver, blood, or brain to yield N-acetylhomotaurinate or related compounds with similar pharmacodynamic properties.

Accordingly, a preferred embodiment provides derivatives of homotaurine and N-acetylhomotaurine effective doses to a patient for treatment of movement disorders. Particularly preferred are derivatives of acamprosate that are readily absorbed from the gastrointestinal tract. Acamprosate is irregularly absorbed from the GI tract, in part due to the polar, hydrophilic character of the acetylhomotaurinate ion. It is well known in the art that certain derivatives of drugs may be absorbed better and more reliably because they are more lipophilic. For example, esters prepared from the acetylhomotaurinate ion would be more lipophilic, and therefore might have greater and more predictable absorption through the membranes of the intestinal mucosa. If such an ester were nontoxic and naturally metabolized in the body, for example, cleaved by enzymes in the blood, liver or the brain, it would be particularly preferred as a vehicle for reliably delivering the acetylhomotaurinate ion to the brain. Furthermore, such derivatives as described above would have, in appropriate dosages, equal or greater efficacy in treating any movement disorder responsive to acamprosate. Generally, any pro-drug with improved delivery of acamprosate would be a preferred means of delivery. Additionally a particularly preferred form of acamprosate would be a derivative of acamprosate with a long half-life. Such a derivative of acamprosate would be clinically superior to acamprosate, because it could be taken once daily, rather than three or four times per day, as is necessary when acamprosate is used. An additional approach to lengthening the half-life of acamprosate or a related medication is to deliver it in a time-release capsule.

In other preferred embodiments, these derivatives are used to treat dyskinetic movement disorders associated with prolonged exposure to neuroleptic medications. Additionally, compositions above can be used to treat tardive dyskinesia in abstinent alcohol abusers who are treated with neuroleptics for concurrent mental disorders, for example bipolar disorder or schizophrenia. More particularly, the present treatments reduce the severity and duration of various related movement disorders.

Another preferred embodiment provides agents that act as GABA agonists and NMDA antagonists as a treatment for focal dystonias. One example of a focal dystonia, blepharospasm, is a target for treatment. As mentioned above, blepharospasm is a condition that involves involuntary forced eye closure. As mentioned above, blepharospasm can occur spontaneously (idiopathic blepharospasm) or can be a form of tardive movement disorder. The eye movement disorder of idiopathic blepharospasm is clinically identical to the one that arises following neuroleptic exposure and therefore might be expected to respond to the same treatments that are efficacious for tardive movement disorders. In fact, both disorders are ameliorated, at least in the short term, by neuroleptic drugs and other dopamine antagonists, and both are responsive to injections of the orbicularis oculi muscles with botulinum toxin (Casey, Neurology, July, 30:690-5, 1980).

The present disclosure demonstrates relief of blepharospasm associated with tardive dyskinesia by treatment with acamprosate, suggesting that acamprosate and related compounds and derivatives with combined action on GABA and NMDA-type glutamate receptors will benefit people with idiopathic blepharospasm and all other focal dystonias, whether spontaneous or induced by exposure to neuroleptic medications.

In one preferred embodiment of this aspect, a pharmaceutical agent is selected from the group of agents that act as GABA-receptor agonists and also act to decrease NMDA receptor function by an indirect or modulatory mechanism such as, in a non-limiting fashion, acamprosate calcium (calcium N-acetylhomotaurinate), other salts of N-acetylhomotaurinate (e.g., magnesium N-acetylhomotaurinate or lithium N-acetylhomotaurinate), acetylhomotaurine base, other homotaurine derivatives with similar pharmacodynamic actions on GABA and glutamate transmission, and pro-drugs that are metabolized in the liver, blood, or brain to yield N-acetylhomotaurinate or related compounds with similar pharmacodynamic actions on GABA and glutamate transmission. In another preferred embodiment, a pharmaceutical agent is selected from the group of agents that have the ability to reduce glutamate-produced excitatory post-synaptic potentials in striatal cells, including acamprosate and the range of similar compounds and pro-drugs described previously). In other preferred embodiments, a combination of two or more pharmaceutical agents is selected such that the combination acts concurrently to augment GABA transmission (particularly via GABA-A receptors) and to attenuate NMDA-type glutamate transmission (e.g., by non-competitive inhibition, or by indirect or modulatory effects on NMDA receptors). A fourth embodiment involves combining such a compound or mixture of compounds with memantine or a similar non-competitive NMDA-receptor blocking agent described in detail below. The combinations may be either mixtures, covalently-bound moieties with combined action, or pro-drugs metabolized in the blood, liver, or brain to release each member of the combination.

Risk factors for TD include advanced age, diabetes, alcoholism and a primary psychiatric diagnosis of a mood disorder rather than schizophrenia. Each of these risk factors also associated with a high prevalence of magnesium deficiency (Durlach, et al., Magnes Res, March, 1998; G'amez et al., Sci. Total. Environ., September 15, 203(3):245-51 1997; Gullestad et al., J Am Coll Nutr, February, 13:45-50, 1994; De Leeuw et al,. Magnes. Res., June, 10:135-41, 1997; Lipski et al, Age Ageing, July, 22:244-55, 1993; Martin et al., J. Trace. Elem. Electrolytes Health Dis, September, 5:203-11, 1991; Shane et al., Magnes. Trace. Elem., 10:263-8, 1991-1992; Zorbas et al., Biol. Trace. Elem. Res., July-August, 58:103-16, 1997). Because people that fit the profile for being at risk for developing TD have an increased risk of magnesium deficiency, I hypothesize that magnesium deficiency (per se) is also a risk factor for tardive dyskinesia and other movement disorders. Therefore, I further assert that magnesium supplementation can alleviate or prevent movement disorders and potentiate the action of other treatments, whether or not the individual treated shows tetany or other signs of magnesium deficiency. (See Case Report 4, in which treatment for a patient with tardive dyskinesia was enhanced by adding a magnesium supplement.)

Risk of developing a movement disorder can be assessed by administering to a patient a sufficient and non-toxic dose of magnesium ion (i.e. a "magnesium load") and subsequently measuring the amount of magnesium ion excreted in the patient's urine. More specifically risk of developing a neuroleptic or dopamine receptor blocker - induced movement disorder can be assessed by performing standard tests of total magnesium status. If magnesium deficiency is present, there is a greater than normal retention of magnesium load, and diminished excretion of magnesium in the urine. If an abnormally low proportion of magnesium is recovered in a 24 hour sample of the patient's urine, the patient is magnesium-deficient and at risk for developing a movement disorder.

The present disclosure demonstrates that supplementation with magnesium can reduce symptoms associated with a simple tic and augment the action of acamprosate in treatment of a simple tic (see Case Report 5). Furthermore, magnesium administered together with acamprosate reduces symptoms associated with simple tic better than either magnesium or acamprosate alone. Together, Cases 4 and 5 suggest that supplementation with magnesium ion may be used to successfully treat other types of movement disorder.

In preferred embodiments, magnesium is used for treatment of movement disorders (e.g., TD, Tourette's Syndrome, and focal dystonias particularly blepharospasm). In addition, magnesium supplementation can be used to reduce the risk of developing a movement disorder. In one preferred embodiment, movement disorders may be prevented by magnesium supplementation. In another embodiment, magnesium supplementation may delay the onset of a movement disorder in a person identified as being at risk for developing a movement disorder. In yet another embodiment, supplementation with magnesium will reduce the symptoms associated with various movement disorders.

Magnesium supplementation will augment the therapeutic effects of other NMDA-type receptor antagonists and down-regulators (see Case Report 5). In one preferred embodiment, magnesium is administered with acamprosate (calcium N-acetylhomotaurine) to treat TD and other movement disorders resulting from neuroleptic drug use, tics, Tourette' syndrome, blepharospasm, other focal dystonias, and the peak-dose dyskinesia of Parkinson's disease. In a particularly preferred embodiment, the magnesium salt of N-acetylhomotaurine and the magnesium salts of those derivatives of N-acetylhomotaurine that similarly enhance GABA transmission and diminish NMDA-glutamate neurotransmission, are effective treatments for movement disorders.

It will be recognized by those skilled in the art that all conditions for which N-acetylhomotaurine is an effective treatment, the magnesium salt of N-acetylhomotaurine, and the magnesium salts of those derivatives of N-acetylhomotaurine that have similar effects on GABA neurotransmission and NMDA-glutamate neurotransmission will also be effective treatments. Alternatively, any magnesium salt may be administered with any salt of those derivatives of N-acetylhomotaurine to treat hyperkinetic and dyskinetic movement disorders. In one non-limiting example, a pill containing the appropriate dose of acamprosate together with the appropriate dose of magnesium may be formulated and administered to a patient with a movement disorder. In other preferred embodiments, an agent that has NMDA antagonist activity and GABA agonist activity is combined with the appropriate dose of magnesium in a pill. In yet another preferred embodiment, an NMDA antagonist is combined with a GABA agonist and an appropriate dose of magnesium in the form of a pill. One of ordinary skill in the art will recognize that the composition of administration is not limited to a pill, but can also be a syrup, an elixir, a liquid, a tablet, a time-release capsule, an aerosol or a transdermal patch.

The ratio of acamprosate to magnesium can be varied to optimize the therapeutic synergy of the two ingredients. Magnesium N-aceytlhomotaurinate (Durlach, *supra;* 1980), with a Magnesium:acetylhomotaurinate ratio of approximately 1:20 by weight, does not optimize the therapeutic effect of the two components. At typical therapeutic dosages of acetylhomotaurinate, the amount of magnesium is too low to have therapeutically-relevant effects on glutamate transmission. In my experience, I have had excellent therapeutic results from combining a 2 gram daily dosage of acamprosate with 1 gram of elemental magnesium, given as a salt or chelate. This combination gives better relief of both TD and tics than 2 grams of acamprosate alone. I have also demonstrated (see Case Report 5 below) that a single dose of 300 mg of magnesium will augment the therapeutic effect of a single 666 mg dose of acamprosate. Allowing for variations in individual response, and variations in the intestinal absorption of both acamprosate and magnesium, I assert that the optimal ratio of mg: acetylhomotaurinate for an individual patient will be somewhere between 1:6 and 1:1. Lower ratios of magnesium to acamprosate are unlikely to boost the therapeutic effect of acamprosate significantly, and higher ratios than 1:1 are likely to produce magnesium toxicity (or at least GI intolerance) at a typical daily acamprosate dose of 2 grams. Although magnesium N-acetylhomotaurinate may be slightly more efficacious than calcium N-acetylhomotaurinate for treatment of tic disorders, in the present application we are effectively increasing the magnesium content of acamprosate and related compounds by administering magnesium ion (as a salt or chelate) in combination with a salt of N-acetylhomotaurinate, because there is a significant benefit to administering a higher ratio of magnesium to acamprosate than is present in the magnesium salt of acamprosate.

The effects of acamprosate are realized within hours after acamprosate administration. This observation is critically important to the hypothesized mechanism of action of acamprosate in the treatment of movement disorders. In 1997, Lidsky et al., (U.S. Patent Number 5,602,150) described the use of taurine and taurine derivatives, including acamprosate, for the *prevention* of tardive dyskinesia in people taking neuroleptic drugs. In a rodent model, animals were given neuroleptics with or without taurine. Over several months, the animals receiving taurine were less likely to develop vacuous chewing movements (VCM), a movement disorder with similarity to TD in humans. The mechanism advanced to explain the effect was a long-term neuroprotective action of taurine, in which taurine blocked the long-term effect of glutamatergic overstimulation of striatal neurons. One of ordinary skill in the art would not expect an agent with neuroprotective activity against glutamate-induced excitotoxicity to necessarily be efficacious in the treatment of severe, established cases of movement disorder, and to produce benefit within hours of administration. Indeed, there are well-known situations in neurology where an effective preventive agent can actually *aggravate* an established case of the condition to be prevented. For example, dopamine agonist antiparkinson drugs may delay the onset of dyskinesia in patients treated with levodopa for Parkinson's disease. Yet, dopamine agonists can aggravate dyskinetic movements once they are established.

Magnesium ions also act as neuroprotective agents, particularly in models of neuronal injury mediated by NMDA-type glutamate receptors (Ema et al., Alcohol, February, 15;95-103, 1998; Greensmith et al., Neuroscience, October, 68:807-12, 1995; Heath et al., J. Neurotrauma, March, 15:183-9, 1998; Hoane et al., Brain. Res. Bull., 45:45-51, 1998; Muir et al., Magnes. Res., March, 11:43-56, 1998; Vanick'y et al., Brain. Res., April, 789:347-50, 1998). However, the virtually immediate benefit of magnesium in the treatment of established movement disorders cannot be based on neuroprotection. Rather, *immediate and direct* effects of magnesium on neural transmission, including glutamatergic transmission, must be involved. In this connection, note that the dosages of magnesium used for neuroprotection in humans usually are well above the 1 gram per day that was the highest dose used here in the treatment of movement disorders. Indeed, magnesium, administered at doses well below those used when magnesium is a single agent can increase the beneficial effects of other treatments, as described herein. Another aspect features agents that act as NMDA antagonists and agonists to improve memory and cognition in humans with TD. A particularly preferred embodiment is to develop agents and preparations for improving cognitive function in patients exhibiting TD, specifically to increase memory, span of concentration, and everyday functional performance in activities particularly dependent upon cognition. These improvements in function are measured both subjectively and objectively. The improvement in memory can be demonstrated by standard neuropsychological tests. The improvement in cognition is demonstrated by performance on neuropsychological tests, including without limitation, the Rey Auditory-Verbal Learning Test, and measurement of Choice Reaction Time, and by subjective indicators of performance at tasks highly dependent on cognitive processes. It will be obvious to one skilled in the art that numerous different neuropsychological tests could be employed to demonstrate that cognitive function improved in patients on a treatment regime that included acamprosate or any of the other above-described agents, including without limitation: other salts of acetylhomotaurine derivatives of homotaurine and acetylhomotaurine with similar pharmacodynamic effects on NMDA-glutamate and GABA neurotransmission, pro-drugs that are metabolized in the blood, liver, or brain to produce acetylhomotaurinate or derivatives with similar pharmacodynamic effects on NMDA-glutamate and GABA neurotransmission, and mixtures of two or more compounds that have, taken together, NMDA-glutamate antagonist and GABA agonist effects. All of these entities may also have neuroprotective actions against glutamate-induced excitotoxic damage, but their virtually immediate beneficial effect on the movement disorders and cognition, which is reversible if the medication is discontinued, cannot be due to such neuroprotective actions.

Another preferred embodiment is the development of agents and preparations for improving tics and, as a consequence, reducing stigma and improving the quality of life for patients with tics or tic disorders such as TS. Yet another embodiment of this aspect is the development of agents and preparations for relieving blepharospasm, and the associated impairment visual function implied by frequent, forceful, involuntary eye closure. A final embodiment of this aspect provides agents and preparations of treating all focal dystonias, whether spontaneous or precipitated by exposure to neuroleptic drugs and other dopamine receptor blockers.

One of ordinary skill in the art will recognize that the present disclosure is not limited to treating TD and other tic disorders with agents that reduce NMDA-type glutamate neurotransmission and increase GABA neurotransmission via direct effects on GABA and NMDA receptors. In addition to direct effects on receptor sites, the agents may modify NMDA-glutamate and GABA transmission through indirect effects on receptors (i.e., via pre-synaptic effects on neurotransmitter release, allosteric modulation of the receptor site, or effects on the intracellular response to the binding of the transmitter to the receptor), presynaptic effects on transmitter release, or any of a variety of mechanisms. It will be obvious to one skilled in the art that a range of derivatives and pro-drugs all should be therapeutically effective. Anything that shares the effects on glutamate and GABA transmission hypothesized to underlie the therapeutic effects of acamprosate is within the scope of the present disclosure. It does not matter how a drug, pro-drug or mixture thereof decreases NMDA-glutamate neurotransmission and increases GABA neurotransmission, only that it improves symptoms associated with TD and tics at tolerably non-toxic (ie., free from toxicity unacceptable side effects) doses.

As discussed previously, the present inventive treatment can be used to treat any movement disorder characterized by any form of abnormal or involuntary movement. Furthermore, the inventive treatment may be used to improve or eliminate symptoms unrelated to movement that are consequences of the movement disorder, for example, cognitive dysfunction or abnormalities of motivation, mood, or impulse control. The latter include anxiety, depression, apathy, aggression, and obsessive compulsive behavior. The basal ganglia, including the striatum, are a point of intersection of motor, cognitive, and emotional circuits. Diseases of the basal ganglia frequently involve cognitive, emotional, behavioral, and motivational changes, as well as motor dysfunction. I expect that drug treatments effective for TD, tics, and other movement disorders might also alleviate some or all of the non-motor symptoms. In general, treatments for diseases of the basal ganglia do have non-motor effects. For example, dopamine agonist antiparkinson drugs not only decrease the rate of movement of patients with Parkinson's disease, they also improve mental processing speed. When the addition of magnesium increases the effect of a drug treatment on the motor manifestations of a movement disorder, it may also increase the effect of that treatment on the non-motor manifestations.

### Memantine

I disclose that memantine, a NMDA-type glutamate receptor antagonist, that also acts as a dopamine agonist, can ameliorate TD as well as related involuntary movements and cognitive symptoms. In particular, I have demonstrated in two patients that memantine can ameliorate blepharospasm associated with a more extensive tardive movement disorder. As discussed above, according to the theory of the present disclosure, NMDA receptor antagonists reduce the severity of the involuntary movements associated with TD. Such an NMDA-type receptor antagonist will likely alleviate focal dystonias and idiopathic focal dystonias, whether or not accompanied by other symptoms of TD, and whether or not related to exposure to neuroleptics or other dopamine receptor antagonists, based on the hypothesis that common response to several therapies implies a common physiology. For example, NMDA receptor antagonists will relieve symptoms of blepharospasm associated with TD, and by extension drug-induced blepharospasm without TD, and idiopathic blepharospasm, which are likely to share a common mechanism, in light of their response to dopamine antagonists, to GABA agonists, and to botulinum toxin injections.

Likewise, treatment with memantine will likely ameliorate symptoms associated with Meige syndrome, which is blepharospasm accompanied by dystonic movements of the neck and lower face. One of the two patients described below (Case Report 1) with TD not only had blepharospasm, but also had dystonic movements of the face and neck, permitting a diagnosis of tardive Meige syndrome. As with blepharospasm alone, Meige syndrome not associated with neuroleptics can be expected to respond at least as well to memantine as tardive Meige syndrome.

The above hypothesis regarding a motor control circuit involving glutamate (via NMDA receptors) suggests that any drug that is an NMDA-type glutamate antagonist, with a relative lack of toxicity at effective doses, can ameliorate blepharospasm, Meige syndrome, and tardive movement disorders. Memantine is a specific example of such a drug for which I offer direct evidence of efficacy in humans (see Case Reports 1 and 6).

As noted above, GABA-A agonists alone are not particularly potent therapies of tics. Therefore, NMDA antagonism is likely a necessary part of the therapeutic effect of acamprosate. Furthermore, NMDA antagonism in itself is sufficient in treatment of tardive dyskinesia and tardive dystonia, suggesting that where tardive movement disorders are concerned, the NMDA antagonist activity of memantine is more important than its dopamine agonism. Evidence disclosed herein suggests that movement disorders, such as tics and Tourette's, will respond in a manner similar to tardive movement disorders to drug therapies having NMDA antagonist activity.

The hypothesis that a common response to several therapies implies common physiologic mechanism in reference to tics ant Tourette's is supported by the fact that: 1) acamproate alleviates tardive dyskinesia, tardive dystonia and tics; and 2) memantine alleviates tardive dyskinesia and tardive dystonia; and 3) both acamprosate and memantine are NMDA-type receptor antagonist. Thus, it is logical to expect that memantine will also be helpful in the treatment of tics, including Tourette's. Indeed, I disclose in the present application that memantine dramatically diminishes dyskinetic movements associated with tic disorders including both single and multiple tics. Furthermore, I propose that memantine and other agents (and congeners and derivatives thereof) with similar pharmacokinetic action, that both (i) decrease NMDA type glutamate neurotransmission, and (ii) increase dopamine receptor neurotransmission are useful in the treatment of a common and severe type of tic disorder, Tourette's syndrome, which is characterized by multiple motor and phonic tics. Case Report 5 demonstrates that a patient with a simple tic experiences a significant reduction in the frequency of the tic upon administration of memantine.

Other examples of drugs with similar effects on NMDA-type glutamate transmission include dextromethorphan, derivatives of memantine and dextromethorphan, and pro-drugs that are metabolized in the liver, blood, or brain to yield biologically active compounds with similar pharmacodynamic properties. Dextromethorphan, like memantine and amantadine, is a NMDA receptor antagonist. In one preferred embodiment, dextromethorphan (and congeners and derivatives thereof), are administered to patients for treatment of movement disorders. There have been no reports of the use of dextromethorphan in the treatment of tardive dyskinesia, dystonia or other movement disorders.

A preferred embodiment of the present invention provides derivatives of memantine and dextromethorphan at effective doses to a patient for treatment of movement disorders. Additionally, particularly preferred forms of memantine and dextromethorphan would be derivatives with longer durations of action, e.g. obtained through longer elimination half-lives. Such derivatives of memantine or dextromethorphan would be clinically superior to memantine or dextromethorphan, because they could be taken once daily, rather than two to four times per day, as is necessary when memantine or dextromethorphan are used. An additional approach to lengthening the duration of action of memantine, dextromethorphan or related medications is to deliver them in a time-release capsule.

In other preferred embodiments, these derivatives of memantine and dextromethorphan are used to treat dyskinesia and dystonia disorders associated with prolonged exposure to neuroleptic medications. Additionally, the compositions described above can be used to treat tardive dyskinesia in patients who continue to be treated with neuroleptics for persistent or chronic mental disorders, for example bipolar disorder or schizophrenia. More particularly, memantine and related compounds reduce the severity and duration of various related movement disorders. Another preferred embodiment of the present invention provides memantine, dextromethorphan and derivatives and congeners thereof as treatment for focal dystonias. One example of a focal dystonia, blepharospasm, is a target for treatment in the present invention.

The present invention demonstrates relief of blepharospasm associated with tardive dyskinesia by treatment with memantine, suggesting that this and related compounds and derivatives with similar action on NMDA-type glutamate will benefit people with idiopathic blepharospasm and all other focal dystonias, whether spontaneous or induced by exposure to neuroleptic medications.

In one preferred embodiment of this aspect of the invention, a pharmaceutical agent is selected from the group of agents that act to decrease NMDA receptor function whether as non-competitive antagonists, ion channel blockers, or modulators of NMDA receptor function. These include, in a non-limiting fashion, memantine, dextromethorphan, and dextrorphan, a derivative of dextromethorphan with known NMDA antagonism and acceptable toxicity for human administration. One of ordinary skill in the art will recognize that the above embodiments include congeners and derivatives of memantine, dextromethorphan and dextrorphan with similar pharmacodynamic actions on glutamate transmission, and pro-drugs that are metabolized in the liver, blood, or brain to yield related compounds with similar pharmacodynamic actions on glutamate transmission. In yet another preferred embodiment, a pharmaceutical agent is selected from the group of agents that have the ability to reduce glutamate-produced excitatory post-synaptic potentials in striatal cells, (including memantine, dextromethorphan and the range of similar compounds and pro-drugs described previously). In other preferred embodiments, a combination of two or more pharmaceutical agents is selected such that the combination acts concurrently to attenuate NMDA-type glutamate transmission (e.g., by non- competitive inhibition, by indirect or modulatory effects on NMDA receptors, or by a combination or sequence of actions). A fifth embodiment involves combining such compounds or mixtures of compounds with a similar non-competitive NMDA-receptor blocking agent described in detail below. The combinations may be either mixtures, covalently-bound moieties with combined action, or pro-drugs metabolized in the blood, liver, or brain to release each member of the combination.

As mentioned previously, risk factors for developing for TD include magnesium deficiency. Thus, I hypothesized that magnesium deficiency is also a risk factor for other movement disorders and that supplementation with magnesium can alleviate or prevent movement disorders alone or in combination with other therapies.

Case Report 2 demonstrates that magnesium administration in combination with acamprosate and memantine augments the therapeutic action of memantine and acamprosate in the treatment of TD. Case Report 5 demonstrates that magnesium administration in combination with memantine alone augments the therapeutic action of memantine alone for treatment of simple tics. Thus, I propose that magnesium will augment treatment when used in combination with memantine or dextromethorphan alone for treatment of tics and Tourette's as it does when used in combination with acamprosate, given the common physiological actions of these compounds. Further support for this hypothesis is the fact that tics and Tourette's, like the tardive movement disorders, are temporarily suppressed by neuroleptics.

Magnesium supplementation can be beneficial whether or not the individual treated shows clinical signs of magnesium deficiency. (See Case Reports 2 and 4 and co-pending Patent Application Serial No. 09/193,892, filed November 18, 1998 entitled "Methods of Treating Tardive Dyskinesia and Other Movement Disorders", for full details. These cases demonstrate that patients whose tardive dyskinesia, which had improved with administration of NMDA receptor antagonists, further improved with the addition of magnesium).

In preferred embodiments of the present invention, magnesium is used to augment the efficacy of memantine, dextromethorphan, or other agents with similar effects upon NMDA-glutamate neurotransmission, in the treatment of movement disorders (e.g., tardive dyskinesia, tardive dystonia, and idiopathic focal dystonias, particularly blepharospasm).

According to the present invention magnesium supplementation will augment the therapeutic effects of other NMDA-type receptor antagonists and down-regulators. In one preferred embodiment, magnesium is administered with memantine to treat TD and other movement disorders resulting from neuroleptic drug use, as well as various focal dystonias. Also according to preferred embodiments of the present invention, magnesium is administered with memantine to treat tics, Tourette's and other tic related disorders. In another embodiment, magnesium is administered with dextromethorphan and memantine to treat TD, tics, Tourettes and other movement disorders resulting from neuroleptic drug use, as well as various focal dystonias. In one final embodiment, magnesium is administered with both memantine and dextromethorphan for treatment of movement disorders.

It will be recognized by those skilled in the art that all conditions for which memantine and dextromethorphan are effective treatments, derivatives and congeners of memantine and dextromethorphan that have similar effects on NMDA-glutamate neurotransmission will also be effective treatments. In one non-limiting example, a pill containing the appropriate dose of memantine together with the appropriate dose of magnesium may be formulated and administered to a patient with a movement disorder. Alternatively, a pill containing the appropriate dose of dextromethorphan together with the appropriate dose of magnesium may be formulated and administered to a patient with a movement disorder. One may also combine appropriate doses of memantine, dextromethorphan and magnesium in a single pill for administration to a patient for treatment of a movement disorder. In another preferred embodiment, an agent that has NMDA antagonist activity is combined with the appropriate dose of magnesium in a pill. In other preferred embodiments, an NMDA antagonist is combined with a dopamine agonist and an appropriate dose of magnesium in the form of a pill. One of ordinary skill in the art will recognize that the composition of administration is not limited to a pill, but can also be a syrup, an elixir, a liquid, a tablet, a time-release capsule, an aerosol or a transdermal patch.

The ratio of memantine and/or dextromethorphan to magnesium can be varied to optimize the therapeutic synergy of the two ingredients. Typically a combination of 5-10 mg of memantine and 250-300 mg of magnesium is administered three times per day to a patient with a movement disorder. This dosage of magnesium is below the pharmacologic doses used for neuroprotection or treatment of eclampsia. The recommended administration regime for dextromethorphan is four times per day at a dose of 30-60 mg with 250-300 mg of magnesium for treatment of movement disorders. One of ordinary skill in the art may experiment with different variations in individual response and intestinal absorption to find the optional ratio of memantine or dextromethorphan to magnesium. The skilled artisan will also recognize that optimal doses may vary if magnesium is administered with dextromethorphan and memantine simultaneously. (Because magnesium is excreted by the kidney, the dose of magnesium would be much lower in patients with renal insufficiency in addition to their movement disorder.)

Memantine, dextromethorphan and magnesium all have been advanced as neuroprotective agents, particularly in models of neuronal injury mediated by NMDA-type glutamate receptors (Representative citations for memantine: (Wenk GL, et al. Behav Brain Res, 83:129-33, 1997 Feb; Kornhuber J et al.: J Neural Transm Suppl, 43:91-104, 1994; Weller M et al.: Eur J Pharmacol, 248:303-12, 1993 Dec 1; Krieglstein J et al. Neuropharmacology, 35:1737-42, 1996). Representative citations for dextromethorphan: (Duhaime AC; et al. J Neurotrauma, 13:79-84, 1996 Feb; Steinberg GK et al. Neurol Res, 15:174-80, 1993 Jun; Britton P et al.: Life Sci, 60:1729-40, 1997). Representative citations for magnesium: Ema et al..Alcohol, February, 15;95-103, 1998; Greensmith et al., Neuroscience, October, 68:807-12, 1995; Heath et al., J Neurotrauma, March, 15:183-9, 1998; Hoane et al., Brain. Res. Bull., 45:45-51, 1998; Muir et al., Magnes. Res., March, 11:43-56, 1998; Vanicky et al., Brain. Res., April, 789:347-50, 1998). However, as mentioned previously for acamprosate, the immediate, short-term benefits of memantine, dextromethorphan, and magnesium for tardive movement disorders cannot be due to their neuroprotective actions, since excitotoxic neuronal damage due to neuroleptics is thought to occur gradually over months to years, while the therapeutic action of the treatments advanced here is virtually immediate.

One of ordinary skill in the art will recognize that the present invention is not limited to treatment with drugs that directly block NMDA-type glutamate receptors. It will be obvious to one skilled in the art that a range of derivatives and pro-drugs all should be therapeutically effective. If a drug decreases NMDA-glutamate transmission by a mechanism other than direct effects on the receptor, or if the active substance is a metabolite of a pro-drug that is administered, it lies within the scope of the presently claimed invention as long as it improves symptoms associated with TD, tardive dystonia, and other dystonias including blepharospasm, at tolerably non-toxic doses (i.e. dosages without severe side effects).

The present invention will now be illustrated by the following non-limiting examples.

### Case Report 1

A 45-year old woman had long-standing TD, originally induced by seven years exposure to amoxapine, an antidepressant drug with neuroleptic effects. The patient's irregularly-rhythmic movements consisted of forced eye blinking (blepharospasm), thrusting of the tongue forward and from side to side, tongue twisting, grimacing, shoulder shrugging, and tensing of the platysma muscles of the neck. (Had the patient's symptoms not been associated with neuroleptic exposure, a subset of her movements could be characterized as the Meige syndrome of oromandibular dystonia with blepharospasm). The patient is a semi-professional musician; the dyskinetic movements were accompanied by significant occupational disability, including difficulty reading music or text and difficulty playing woodwind instruments. Much of her reading impairment was due to frequent involuntary blinking and eye closure. She had impaired attention, concentration and memory compared with her performance before the onset of TD. She had significant fatigue, and usually required rest at some point during each day. The patient was diagnosed with TD by a board-certified neurologist with extensive experience in evaluating neuroleptic-induced side effects.

The patient's dykinesia and dystonia worsened after the amoxapine was discontinued. Palliative treatment with alprazolam (an anxiolytic and GABA agonist via modulation; dosage 0.25 mg four times a day) and trihexyphenidyl (an anticholinergic antiparkinson drug that inhibits dopamine re-uptake at synapses; dosage 2 mg twice a day) was prescribed by another physician. This combination produced minimal improvement. The patient began treatment with me in the winter of 1992 and was maintained on trihexyphenidyl for an additional 18 months. Trihexyphenidyl was then discontinued without a change in her involuntary movements. During 1993, alprazolam was increased to 0.5 mg four times a day, to treat mild symptoms of anxiety; the change in dosage had no detectable effect on the patient's involuntary movements. Treatment trials with buspirone, sertraline, verapamil, and vitamin E in 1992 either produced little benefit or were not tolerated at doses that only slightly reduced her involuntary movements. None of these drugs significantly improved the patient's everyday function, i.e., her performance at reading text or music, her stamina or her ability to concentrate. The first drug that provided significant and sustained benefits was nimodipine, a blocker of L-type *calcium* channels, that indirectly reduces dopaminergic activity (Bonci et al; J. Neurosci., September 1, 18(17):6693-703 1998)

Beginning in 1993, nimodipine was administered at a dosage of 30 mg four times a day. Initially, her other medications were maintained unchanged. This regime reduced the patient's involuntary movements by about 50%. Unfortunately, the patient experienced adverse effects, including dizziness, lightheadedness, and palpitations. Also, she had no symptomatic improvement in cognitive function. There was a meaningful improvement in her ability to read and to play music. However, even with this improvement, she could read text or music for no more than 30 minutes at a time, before fatigue or blepharospasm prevented her from continuing.

In 1995, memantine came to my attention as a relatively non-toxic NMDA receptor antagonist. In view of my hypothesis about the pathophysiology of tardive dyskinesia, I thought that memantine might be beneficial in its treatment. Nimodipine was discontinued, and the patient was begun on memantine at a dosage of 10 mg twice a day. The involuntary movements of the patient's TD were reduced within 24 hours of administration of memantine, to a substantially greater degree than had been observed with nimodipine. Adverse effects included a sense of mild intoxication. Adjustments to the therapeutic regime were made such that the drug was reduced to 5 mg three times a day, with the result that the therapeutic benefits were maintained without perceptible side effects. In addition, the patient reported improved energy, attention, and concentration. Memantine was the patient's primary treatment for TD for the next 1 1/2 years, until I became aware of acamprosate as an indirect NMDA antagonist with the added benefit of GABA agonism.

Prior to treatment with acamprosate, the patient's involuntary movements (on an optimal dose of memantine) consisted of eye blinking, puckering of the cheeks, writhing of the tongue and tensing of the platysma. These involuntary movements were usually mild and occasionally moderate in intensity. The movements had been substantially more severe in the past, but had been reduced significantly during the two-year course of treatment. Moreover, the patient's involuntary movements were accompanied by mild but definite cognitive impairment. The patient's most prominent cognitive symptom was difficulty sustaining concentration long enough to read more than a few pages of text.

The patient was taken off of memantine and treated with acamprosate at a dose of 333 mg four times a day. On acamprosate, the patient's involuntary movements (forced eye blinking (blepharospasm), thrusting of the tongue forward and from side to side, tongue twisting, grimacing, shoulder shrugging, and tensing of the platysma muscles of the neck) became imperceptible.

In addition, the patient's cognitive function improved significantly when measured both subjectively and objectively. On acamprosate, the patient was able to sustain concentration for prolonged periods. For example, she could now read a book for over an hour at a time, with good recall of what she had read. The patient's cognitive improvement was also assessed using formal neuropsychological measures. The patient was tested while on the drug, then taken off of the drug and tested two days later. On the drug, the patient was able to recall 13 of the 15 items after a short delay as well as 13 of the 15 items after a long delay, as measured by the Rey Auditory Verbal Learning Test. This was in comparison to the patient's ability while off the drug to recall only 7 of the 15 items after a short delay as well as 8 of the items after a long delay in tests performed. In addition, the patient was able to recognize all 15 of the items while on acamprosate but while off the drug (and while having been off of memantine for over 2 months) the patient could only recognize 10 of the items. The order of testing would give the advantage of familiarity to the off-drug condition. Nonetheless, the difference in favor of the on-drug condition was substantial.

Comparison with other neuropsychological tests demonstrated that the improved cognitive findings shown while the patient was on acamprosate were not explained by a nonspecific lack of effort or to concentration during off-drug condition. These additional tests, which reflect basic attention and psychomotor speed, showed that the patient actually had slightly better results *off* acamprosate. The tests showing such results included Simple Reaction Time, the Trail Making Test (both parts) and the Paced Auditory Serial Addition Test (PASAT). Choice reaction time, a test requiring both basic attention and concentration on a specific task that must be kept in mind, was slightly better *on* acamprosate, consistent with the hypothesis that general cognitive function, as opposed to simple attention, improves with acamprosate treatment.

The following tables report on the results of the neuropsychological tests (Drug I is memantine and Drug II is acamprosate):

**TABLE 1:**

| REACTION TIME, PSYCHOMOTOR SPEED, & MOTOR FUNCTIONING FOR DRUG I (MEMANTINE) AND DRUG II (ACAMPROSATE) | | | | | |
|---|---|---|---|---|---|
| TESTS | 2/23/94 | 2/23/96 ON DRUG I | 4/8/96 OFF DRUG I | 9/23/97 ON DRUG II | 9/25/97 OFF DRUG II |
| Simple Reaction Time^{a} | | | | | |
| 1500 Green | NA | 212 msec | 332 msec | 261 msec | 234 msec |
| 1500 Red | NA | 224 msec | 276 msec | 264 msec | 241 msec |
| 500 Green | NA | 284 msec | 343 msec | 286 msec | 272 msec |
| 500 Red | NA | 266 msec | 382 msec | 272 msec | 237 msec |

| Choice Reaction Time^{a} | | | | | |
|---|---|---|---|---|---|
| 1500 Green | NA | 365 msec | 542 msec | 408 msec | 442 msec |
| 1500 Red | NA | 422 msec | 643 msec | 379 msec | 435 msec |
| 500 Green | NA | 362 msec | 603 msec | 382 msec | 425 msec |
| 500 Red | NA | 421 msec | 557 msec | 426 msec | 413 msec |

| PASAT^{a} | | | | | |
|---|---|---|---|---|---|
| 2.4 sec ISI errors | 17/49 | 13/49 | 15/49 | 4/49 | 0/49 |
| 2.0 sec ISI errors | 17/49 | 17/49 | 21/49 | 1/49 | 1/49 |
| 1.6 sec ISI errors | 11/49 | 21/49 | 22/49 | 11/49 | 4/49 |
| 1.2 sec ISI errors | 17/49 | 28/49 | 25/49 | 13/49 | 11/49 |
| Digit Symbol^{b} | NA | 34 | 20 | NA | NA |

| Trails A | | | | | |
|---|---|---|---|---|---|
| Seconds^{a} | 25" | 28" | NA | 20" | 16" |
| Errors^{a} | 1 0 | | NA | 0 | 0 |

| Motor Functions | | | | | |
|---|---|---|---|---|---|
| Grooved Functions sec.^{a} DH=right | DH=68" | DH=71" | NA | DH=61" | DH=59" |
| | NDH=8 2" | NDH=70" | | NDH=70" | NDH=76" |
| Finger Tapping^{b} | DH=58. 8 | DH=59.3 | NA | NA | NA |
| | NDH=4 1.6 | NDH=48.5 | | | |
| Grip Strength^{b} | NA | DH=17.7 | NA | NA | NA |
| | | NDH=21.7 | | | |

| | | | | | |
|---|---|---|---|---|---|
| Note:^{a} lower score indicative of better performance ^{b} higher score indicative of better performance | | | | | |

**TABLE 2:**

| EXECUTIVE, ATTENTION, VISUOCONSTRUCTIONAL & VISUAL MEMORY TASKS FOR DRUG I (MEMANTINE) AND DRUG II (ACAMPROSATE) | | | | | |
|---|---|---|---|---|---|
| TESTS | 2/23/94 | 2/23/96 ON DRUG I | 4/8/96 OFF DRUG I | 9/23/97 ON DRUG II | 9/25/97 OFF DRUG II |
| 1. Trails B | | | | | |
| Seconds^{a} | 56" | 118" | NA | 43" | 39" |
| Errors^{ab} | 0 | 0 | NA | 0 | 0 |

| Verbal Fluency | | | | | |
|---|---|---|---|---|---|
| Letter | NA | Total = 70 | NA | NA | NA |
| (CFL)^{b} | | Per = 2^{b} | | | |
| Category (Animals)^{b} | NA | Total = 25 | NA | NA | NA |
| | | Per = 0^{b} | | | |

| Figural Fluency | | | | | |
|---|---|---|---|---|---|
| Unique Designs^{b} | NA | 124 | 99 | NA | NA |
| Perseverations^{a} | NA | 8 | 4 | NA | NA |

| 2. CPT - with conditions (Vigilance) | | | | | |
|---|---|---|---|---|---|
| Commission errors^{a} | 0 | NA | NA | 0 | 0 |
| Omission errors^{a} | 0 | NA | NA | 0 | 0 |
| Wrong^{a} | 3 | NA | NA | 3 | 0 |
| Correct^{b} | 50/50 | NA | NA | 100/100 | 100/100 |

| 3. Rey-Osterrieth Complex Figure | | | | | |
|---|---|---|---|---|---|
| Copy Presence & Accuracy^{b} | NA | 20 | 17 | NA | NA |
| Copy Organization^{b} | NA | 5 | 4 | NA | NA |
| Immediate Retention^{b} | NA | -55 | -47.1 | NA | NA |
| Delayed Retention^{b} | NA | -11.1 | 22.2 | NA | NA |

| | | | | | |
|---|---|---|---|---|---|
| Note: ^{a} lower score indicative of better performance ^{b} higher score indicative of better performance | | | | | |

**TABLE 3:**

| MEMORY TESTING FOR DRUG I (MEMANTINE) AND DRUG II (ACAMPROSATE) | | | | | |
|---|---|---|---|---|---|
| TESTS | 2/23/94 | 2/23/96 ON DRUG I | 4/8/96 OFF DRUG I | 9/23/97 ON DRUG II | 9/25/97 OFF DRUG II |
| California Verbal Learning Test 16-items | | | | | |
| List A 1-5 total (80 max)^{b} | NA | 53 | 40 | NA | NA |
| List A Trial 1^{b} | NA | 7 | 6 | NA | NA |
| List A Trial 5^{b} | NA | 13 | 9 | NA | NA |
| List B^{b} | NA | 7 | 5 | NA | NA |
| Short-Delay Free Recall^{b} | NA | 10 | 4 | NA | NA |
| Short-Delay Cued Recall^{b} | NA | 13 | 9 | NA | NA |
| Long-Delay Free Recall^{b} | NA | 12 | 7 | NA | NA |
| Long-Delay Cued Recall^{b} | NA | 15 | 8 | NA | NA |
| Perseverations^{a} | NA | 23 | 4 | NA | NA |
| Intrusions^{a} | NA | 6 | 0 | NA | NA |
| Recognition Hits^{b} | NA | 16 | 14 | NA | NA |
| False Positives^{a} | NA | 3 | 0 | NA | NA |

| Rey-Auditory Verbal Learning Test 15-items | | | | | |
|---|---|---|---|---|---|
| List A 1-5 toal (75 max)^{b} | NA | NA | NA | 63 | 57 |
| List A Trial 1^{b} | NA | NA | NA | 10 | 9 |
| List A Trial 5^{b} | NA | NA | NA | 14 | 14 |
| List B^{b} | NA | NA | NA | 8 | 9 |
| Short-Delay Free Recall^{b} | NA | NA | NA | 13 | 7 |
| Long-Delay Free Recall^{b} | NA | NA | NA | 13 | 8 |
| Perseverations^{a} | NA | NA | NA | 5 | 0 |
| Intrusions^{a} | NA | NA | NA | 0 | 3 |
| Recognition Hits^{b} | NA | NA | NA | 15 | 10 |
| False Positive^{a} | NA | NA | NA | 1 | 2 |

| | | | | | |
|---|---|---|---|---|---|
| Note: ^{a}lower score indicative of better performance ^{b} higher score indicative of better performance | | | | | |

In addition to increased cognitive ability, the patient also experienced an increase in stamina while taking acamprosate. Prior to beginning the acamprosate regime, the patient was fatigued by the end of the afternoon, requiring rest in order to be alert in the evening. This fatigue was significantly decreased while on the acamprosate regime, with a corresponding improvement in fatigue-related cognitive function. On acamprosate, the patient no longer needed to rest during the day in order to be alert and active during the evening.

To verify that the acamprosate was related to the patient's improvement in controlling movement disorders, cognitive function and stamina, the patient was removed from the acamprosate regime (as well as the memantine regime) for a period of four weeks. During the initial two-week period off acamprosate, the patient's involuntary movements gradually returned to her pre-acamprosate, off-memantine baseline. (While the patient's off-drug baseline was less severe than it was when she started on memantine two years earlier, her movements still were severe enough to interfere significantly with her everyday functioning.) From that point on, until acamprosate was re-instituted, she showed continual mild-to-moderate grimacing, tensing of the platysma, and forced eye closure. These involuntary movements worsened still further during periods of stress or fatigue. Moreover, the patient fatigued much more easily, to a degree that noticeably reduced her everyday functioning. Subjectively, the patient reported that concentration and memory both decreased.

Within two days of re-instituting treatment with acamprosate, the patient reported that her energy, stamina, concentration and memory had improved to the level experienced during her prior treatment with acamprosate. Two months after reinstitution of acamprosate, the patient's involuntary movements were absent except for very mild movements during times of stress.

In July 1998. this patient participated in a trial of magnesium supplementation as an adjunct to her treatment with acamprosate. During a 7 day baseline period on Campral Cacamprosate 333 mg four times a day plus alprazolam 0.25 mg four times a day, she noted six episodes of involuntary movements involving the face and neck, 2 moderate and 4 mild. For the following 10 days she added 250 mg three times a day of chelated magnesium. During the period of magnesium supplementation, she noted no involuntary movements.

On a separate occasion, the patient was treated again with memantine. Subjectively, the patient reported that her everyday function was improved to a greater extent during treatment with memantine than that experienced during treatment with nimodipine. She was able to read or to play her instrument for longer periods with less of a need for rest during the day. Objectively, her cognitive functioning, including attention span, concentration span and memory improved as indicated by neuropsychological testing.

Discontinuation of memantine resulted in obviously increased dyskinesia within 24 hours, to the point that the movements interfered with reading and musical activities, and caused the patient subjective distress. After re-starting memantine, involuntary movements were reduced to their previous on-treatment level within 24 hours.

The patient's excellent response to memantine supported my hypothesis that NMDA- receptor blockers might be helpful in tardive dyskinesia. To further that hypothesis, the patient was treated with dextromethorphan, an NMDA-receptor blocker thought to act at a different site on the NMDA receptor than that observed with memantine. Moreover, dextromethorphan is not a dopamine agonist like memantine or amantadine. Memantine was discontinued and the patient was started on dextromethorphan, 30 mg four times a day. Within 24 hours, the patient's dyskinetic involuntary movements were reduced to the levels seen while the patient was on memantine. However, the patient felt sedated, and felt that her attention span was shorter and her concentration worse than that experienced while on memantine.

Administration of dextromethorphan was continued for one week and reduction of the involuntary movements continued throughout this period. Increased dyskinesia was seen shortly after discontinuation of dextromethorphan administration. Again, memantine was administered, with the result that the dyskinetic movements were reduced to the same extent as during the previous administration of memantine.

### Summary:

This example demonstrates that efficacious treatments for TD include memantine and acamprosate. Both treatments improve cognition and function as well as involuntary movements. Furthermore, both memantine and acamprosate relieve blepharospasm and Meige syndrome associated with more extensive tardive movement disorders. Finally, oral magnesium administration, given together with acamprosate at a ratio of 1:1.8 by weight, augments the therapeutic effect of acamprosate on the involuntary movements ofTD.

### Case Report 2

A 79-year old woman had long-standing TD following decades of treatment with the neuroleptic drug perphenazine. Her involuntary movements comprised bilateral chorea of the upper extremities, plus writhing of the tongue and tongue-biting. Both of the latter movements led to a very sore tongue. In addition, the patient experienced impairment of her short-term memory, which was attributed primarily to cerebrovascular disease.

Following treatment with memantine the patient's voluntary movements improved, but continued at a mild-to-moderate level. She also continued to have a sore tongue. Her cognitive symptoms did not improve. In addition to memantine, the patient regularly took antiepileptic drugs (gabapentin and lamotrigine), antiplatelet agents (aspirin and ticlopidine), as well as medications for hypertension, glaucoma and gastrointestinal symptoms (isosorbide mononitrate, metoprolol, timolol eye drops and olsalazine). These various drugs did not affect the patient's involuntary movements or cognitive symptoms; there was no noticeable change in either one at the time that each of the above-mentioned drugs was instituted.

The patient was placed on a treatment regime that included administration of 666 mg of acamprosate, three times daily. In this case, acamprosate was *added* to the patient's regimen, which continued to include memantine. Once the patient began taking acamprosate, her chorea and tongue-biting stopped completely, and the writhing movements of the tongue diminished substantially. Subjectively, the patient's memory improved to the extent that her long-term bridge partner stated that patient was noticeably better at remembering cards during the play of duplicate bridge. Despite past evidence from formal testing that the patient had impaired short-term memory, she performed normally on a two-sentence memory task, which involved testing the patient's recall ability using two sentences containing 13 separate details. On the two-sentence memory task, within three attempts the patient was able to recall 9 details and, using a multiple choice format, was able to recall a total of 11 details. Recall of 9 details on the third attempt would be normal for a middle-aged adult, let alone one in her 80s at the time of testing.

After a full year on memantine and acamprosate, the memantine was discontinued, with little change in the patient's symptoms. On acamprosate 666 mg three times a day, persistent symptoms included mild choreatic movements on the hands, mild involuntary movements of the tongue and jaw, and soreness of the tongue disproportionate to the visible involuntary movements.

Magnesium oxide, 250 mg three times a day, was added, each dose being taken together with the acamprosate. The movements and the tongue soreness improved further. The effect was definite: movements worsened when magnesium oxide was stopped and improved when it was restarted. After a month on magnesium, the dosage of acamprosate was increased to 666 mg four times a day, with 250 mg of magnesium oxide given together with each dose. On this regimen, the tongue movements and tongue soreness were completely eliminated. The only residual sign of TD was a mild degree of involuntary movement of the hands.

### Summary:

Magnesium and acamprosate are both efficacious treatments of tardive dyskinesia when administered alone. More specifically, Case Report 2 demonstrates that acamprosate can improve both the involuntary movements associated with TD as well as the associated cognitive impairment, in a patient in whom memantine improves involuntary movements but not cognition. Furthermore magnesium, when administered with acamprosate can augment the efficacy of acamprosate in the treatment of TD. In this case, the combination of acamprosate and magnesium was efficacious at an magnesium:acamprosate ratio or 1:2.66. It is logical to infer that treatment with memantine and magnesium would be better than memantine treatment alone.

### Case Report 3

A 56-year old female professor of nursing had Parkinson's disease since her late 30s. The patient's Parkinson's disease was treated using levodopa/carbidopa and bromocriptine. The patient's profession required a high level of mobility and physical effort, but taking a sufficient dosage of the levodopa/carbidopa to allow adequate physical functioning at work resulted in the patient demonstrating severe peak-dose dyskinesia. The patient's manifestations of peak-dose dyskinesia consisted of writhing movements of the upper trunk, jerky lateral and rotatory movements of the neck, and chorea of both upper extremities. The patient accepted these involuntary movements because lower dosages of levodopa-carbidopa left her too rigid and hypokinetic to perform her job.

Prior to beginning treatment with acamprosate, the patient was on an antiparkinson treatment regime that consisted of 1 mg of pergolide three times a day, 5 mg of selegiline twice a day, and a combination of levodopa/carbidopa consisting of 550-600 mg of levodopa and 125-150 mg of carbidopa administered in divided doses. Concurrent medications that did not appear to affect her Parkinsonism or dyskinesia consisted of bethanecol, sertraline, carbamazepine, conjugated estrogens and medroxyprogesterone. (As with the additional medications mentioned in Case 2, there had been no noticeable change in the patient's Parkinsonism or dyskinesia after the introduction of each of the drugs listed.) The patient also received 10 mg of memantine three times a day, which had previously reduced her dyskinetic movements from severe to mild-to-moderate.

The patient began acamprosate as an addition to the antiparkinson regime described above. Initially, the patient received 666 mg of acamprosate administered three times a day. Two weeks later the regime was adjusted such that the patient received 333 mg of acamprosate four times a day, taking one 333 mg pill with each daytime dose of 100 mg levodopa and 25 mg carbidopa. The patient's bedtime does of controlled-release levodopa-carbidopa (200 mg of levodopa and 50 mg of carbidopa) was continued, but were given without acamprosate. As soon as acamprosate was added to her regimen, the patient's severe peak-dose dyskinesia was reduced from moderate to mild intensity, and there were periods of up to two hours following each dose during which there was no dyskinesia at all. There was no decrease in the efficacy of the levodopa/carbidopa treatment of her hypokinesia and rigidity. On acamprosate, the patient experienced longer periods of good motor function, and she now had no periods at all where her motor function was inadequate for work or social activity. The reduction of the dyskinesia to a minimal level led to a substantial improvement in purposeful motor function of the upper extremities. To confirm that the patient's improvement was due to the administration of acamprosate, the patient was taken off the acamprosate. Within one day of stopping acamprosate, the patient's dyskinetic movements were as severe as they had been before acamprosate was first given. Upon re-instituting acamprosate, the patient experienced an immediate reduction in her dyskinetic movements. During the period off acamprosate, an attempt was made to replace acamprosate with baclofen (a GABA-receptor agonist) at a total daily dose of 30 mg, and then with baclofen at a total daily dose of 60 mg. These doses of baclofen were high enough to produce sedation and nausea, but they had no beneficial effect on the patient's dyskinesia. Additional improvement in the dyskinesia was subsequently obtained by replacing the pergolide with 1 mg. of pramipexole administered three or four times a day.

Several months later, magnesium (300 mg elemental magnesium, as a mixed chelate), was added to the regimen. It was taken three times a day, together with a one of the patient's regular doses of levodopa/carbidopa. There was an immediate reduction of the severity of dyskinesia. To establish whether the improvement was due to magnesium, the magnesium was stopped after several weeks. Within 2 days, the dyskinesia was definitely worse.

### Summary:

Case Report 3 demonstrates that memantine and acamprosate can ameliorate the peak dose dyskinesia of treated Parkinson's disease. The efficacy of memantine and acamprosate in the treatment of the peak dose dyskinesia of Parkinson's disease can furthermore be augmented by coadministration of magnesium at a ratio of 1:1.48 by weight with acamprosate.

### Case Report 4

A 37-year old man had extremely severe tardive dyskinesia and dystonia, as a result of over 15 years of treatment of bipolar disorder with lithium and an assortment of neuroleptics. His involuntary movements consisted of forced extension of the trunk, torsion of the lower legs, plantar flexion of the left foot, chorea of both arms, writhing of the tongue and grimacing. In addition, he had profuse sweating associated with the involuntary movements. To sit still in a chair, he had to forcefully grip both arms. In the chair, forced extension of the trunk practically lifted him out of the chair. His trunk and leg movements led to impaired balance, with a staggering gait and frequent near-falls. The continual severe movements were associated with impairment in concentration, which made his work less efficient. By virtue of talent and intelligence, however, he was able to work competitively as a software engineer. Because his bipolar disorder remained an active problem, continued neuroleptic treatment was necessary to maintain his mental health. He was maintained on lithium carbonate 300 mg three times a day, and risperidone 4 mg per day.

His movement disorder had been treated with benzodiazepines, anticholinergics, and dopamine agonists, all without meaningful benefit. He was then treated with acamprosate, first at a dosage of 333 mg three times a day, and then at a dosage of 666 mg three times a day. Acamprosate therapy was then augmented with magnesium sulfate, 300 mg three times a day. After several weeks, memantine 10 mg three times a day was added, but memantine was discontinued after a few days because it aggravated his movement disorder.

At one point during his treatment, the patient ran out of acamprosate, and was without it for three days. After 24 hours off acamprosate, his movement disorder returned to its (severe) baseline. 72 hours after resuming acamprosate, he regained his previous level of benefit.

The patient maintained a weekly log of symptoms, which is reproduced here as Table 4. Table 4 shows that:
1) Acamprosate therapy was associated with improvement in all of his symptoms. For several of his symptoms - trunk movement, balance, and sweating 666 mg of acamprosate three times a day of acamprosate was more efficacious than 333 mg three
2) The addition of magnesium was associated with further improvement in several symptoms,
   i.e., movements of the face and tongue, neck and limbs;
3) Benefits of acamprosate increased with continued therapy;
4) Mental function, as indicated by subjective memory, improved along with the involuntary movements;
5) The addition of memantine aggravated the involuntary movements.

The patient's self-ratings understate the degree of improvement noted by three physicians (two neurologists and one psychiatrist) who examined the patient before and after treatment with acamprosate and magnesium. Before treatment, he was unable to sit in a chair without gripping the arms, writhing and rocking wildly. After treatment, he was able to walk across a room carrying a cup of coffee and not spilling any.

**TABLE 4:**

| **PATIENT SELF-REPORT OF TD TREATMENT EFFECTS - CASE 4** | | | | | | | | | | |
|---|---|---|---|---|---|---|---|---|---|---|
| **Regimen** | | | | | | | | | | |
| 1. Acamprosate 333 mg three times a day | | | | | | | | | | |
| 2. Acamprosate 666 mg three times a day | | | | | | | | | | |
| 3. Acamprosate 666 mg three times a day + Magnesium sulfate, 300 mg three times a day | | | | | | | | | | |
| 4. Acamprosate 666 mg three times a day + memantine 10 mg three times a day | | | | | | | | | | |

| **Symptoms** | | | | | | | | | | |
|---|---|---|---|---|---|---|---|---|---|---|
| Severity of face and tongue movements (10 is worst) | | | | | | | | | | |
| Severity of trunk movements (10 is worst) | | | | | | | | | | |
| Difficulty maintaining balance (10 is worst) | | | | | | | | | | |
| Sweating (10 is worst) | | | | | | | | | | |
| General well being (10 is best) | | | | | | | | | | |
| Memory and concentration (10 is best) | | | | | | | | | | |

| **Week Number** | 0 | 1 | 2 | 3 | 4 | 5 | 6 | 7 | 8 | 9 |
|---|---|---|---|---|---|---|---|---|---|---|
| Regimen # | baseline | 1 | 2 | 3 | 3 | 3 | 3 | 3 | 4 | 3 |

| **Scale** | | | | | | | | | | |
|---|---|---|---|---|---|---|---|---|---|---|
| Face/tongue | 7 | 5 | 7 | 6 | 4 | 2 | 3 | 3 | 6 | 4 |
| Neck | 7 | 7 | 7 | 6 | 5 | 5 | 4 | 3 | 6 | 4 |
| Trunk | 9 | 8 | 6 | 6 | 4 | 4 | 4 | 5 | 7 | 4 |
| Limbs | 8 | 6 | 7 | 6 | 4 | 2 | 3 | 5 | 7 | 4 |
| Balance | 7 | 7 | 5 | 6 | 6 | 4 | 4 | 4 | 6 | 4 |
| Sweating | 10 | 8 | 5 | 6 | 5 | 3 | 4 | 4 | 5 | 4 |
| Well-being | 7 | 8 | 8 | 8 | 9 | 9 | 9 | 9 | 7 | 9 |
| Memory | 8 | 9 | 9 | 9 | 9 | 9 | 9 | 9 | 9 | 9 |
| Side effects* | | 7 | 7 | 9 | 9 | 9 | 9 | 9 | 7 | 9 |

| | | | | | | | | | | |
|---|---|---|---|---|---|---|---|---|---|---|
| Side Effects(10 is none) * The only significant side effect was nausea and vomiting, which the patient experienced for the first day after starting acamprosate, the first day after increasing the dose of acamprosate, and the first day after adding memantine. | | | | | | | | | | |

### Summary:

Acamprosate is efficacious in the treatment of severe tardive dyskinesia and dystonia. Administration of magnesium with acamprosate enhances the therapeutic action of acamprosate in the treatment of severe TD and tardive dystonia. In the case reported, a good effect was obtained at a magnesium:acamprosate ratio of 1:2.22. Memantine, though often effective in the treatment of tardive dyskinesia, actually can aggravate it in certain individuals, such as the one described in Case 4. Treatment with acamprosate, with or without magnesium, can help alleviate a movement disorder that is aggravated by memantine. Additionally, this case report demonstrates that acamprosate, administered with or without magnesium, can relieve involuntary movements and other symptoms in patients with tardive movement disorders who continue to receive neuroleptics for their mental disorder.

Finally, Case 4 illustrates the point that a treatment that prevents the development of involuntary movements in an animal model of TD (i.e. memantine, see Andreassen et al. *supra)* may be of no benefit at all in treating humans with established TD.

### Case Report 5

A 46 year old man had simple tic of the neck that involved forceful extension and rotation of the neck to the right. The tic had started in the context of therapy of depression with dextroamphetamine and pramipexole, a dopamine agonist drug. The tic occurred from 20-50 times per hour, with greater frequency when he was tired or under stress.

He was initially treated with 666 mg of acamprosate three times a day. Within 24 hours after the start of acamprosate therapy, the frequency and severity of the tic decreased dramatically, to a rate of less than 5 per hour. The patient often was free of tics completely for 2 to 3 hours after each dose of acamprosate, after which time the tic would very gradually return. The dose was then raised to 666 mg four times a day. On this dose, rates of more than 5 per hour occurred only under unusually stressful circumstances, and there were frequent tic-free periods of 4 hours or more. If acamprosate was omitted for a full day, the frequency of tics rapidly increased, to over 10 an hour. On a second day without acamprosate, the rate of tics was again over 20 per hour.

He then added chelated magnesium, at a dosage of 300 mg of elemental magnesium 3 times a day. With magnesium supplementation, the average tic frequency dropped to 6 hour or less. When 666 mg of acamprosate was given three times a day was given together with magnesium 300 mg three times a day, the usual tic-free period after each acamprosate dose increased from approximately 3 hours to approximately 5 hours.

Acamprosate treatment was later replaced with memantine, 10 mg twice a day, for two doses with supplemental magnesium (300 mg). Memantine with magnesium resulted in a tic-free interval of 6-7 hours. Next, the memantine was administered, 10 mg twice a day for two doses without supplemental magnesium. Administration of memantine without magnesium produced a tic-free period of only 4-5 hours beginning about 30 minutes after a dose.

### Summary:

Acamprosate is efficacious in the treatment of a simple tic. The efficacy of acamprosate is enhanced by concurrent administration of magnesium. In this case, a good effect was obtained at a magnesium:acamprosate ratio of 1:2.22. By extension, acamprosate should be efficacious in the treatment of multiple tics and Gilles de la Tourette syndrome. Furthermore, this study demonstrates that memantine is an efficacious treatment for tics and the efficacy of memantine is enhanced by concurrent administration of magnesium in a manner similar to that of acamprosate.

### Case Report 6

A corresponding physician the United Kingdom recently reported to me on the treatment of a 47-year old woman with chronic schizophrenia and severe tardive dyskinesia. As in Case 1, the patient's involuntary movements included severe blepharospasm. In addition, she had involuntary rhythmic peri-oral movements, and chorea-like movements of both hands, like the patient in Case 2. She had no cognitive complaints, nor were cognitive abnormalities noted on routine psychiatric examination.

The patient had developed symptoms of paranoid schizophrenia in 1991, at age 40. The symptoms of psychosis included auditory hallucinations, bizarre delusions, and persecutory fears. She was started on oral haloperidol as an outpatient in July 1992 and had an acute dystonic reaction to the drug. She was subsequently hospitalized and stabilized on fluphenthixol decanoate, a depot neuroleptic given by intramuscular injections. Symptoms of TD developed in November, 1994, after 28 months of neuroleptic therapy. Switching the patient to an atypical neuroleptic, olanzapine or risperidone, did not eliminate her TD. Beginning in October 1997 the patient was treated for her schizophrenia with 2 mg of risperidone alone. On this modest dose of an atypical neuroleptic, she had severe symptoms of TD for which she eagerly sought treatment.

Memantine was started on November 28, 1997 at a dose of 5 mg per day, increased after 7 days to 5 mg twice a day, and after another 7 days to 5 mg three times a day. After the first two weeks of memantine treatment (an on 5 mg twice a day) there was marked improvement in blepharospasm, though the movements started to return just before the second dose of the day was due. Two weeks later, on 5 mg three times a day, improvement was more sustained, with virtually no involuntary movements noted at the peak of a given dose of memantine, and only mild movements noted when a dose was due. Further dosage increases were attempted to completely abolish the involuntary movements. The maximum dose attainable without side effects was 10 mg twice a day; above that level the patient had complaints of dizziness. That dose of memantine was maintained through May of 1997. At that point, after 6 months of treatment with memantine, the patient had no blepharospasm or limb chorea, and only mild peri-oral movements.

In May 1998 the patient was started on acamprosate, in pursuit of complete elimination of her involuntary movements. Initially, acamprosate 333 mg three times a day was added to memantine 10 mg twice a day. With the addition of acamprosate, peri-oral movements were eliminated, and the patient was essentially free of involuntary movements. Memantine was discontinued in August 1998; the patient continued free of involuntary movements on acamprosate alone.

### Summary:

Both memantine and acamprosate can alleviate the involuntary movements of TD in patients with chronic schizophrenia who continue to require neuroleptic therapy. Both drugs can relieve severe neuroleptic-induced blepharospasm. Acamprosate can relieve involuntary movements of TD that do not respond to memantine at doses tolerated by the patient. The response of drug-induced blepharospasm to these two agents suggests that memantine and acamprosate will be helpful in the treatment of idiopathic (spontaneous) blepharospasm. By extension, they can be expected to be useful in the treatment of other focal dystonias.

### Discussion

The patients discussed in the cases presented all exhibited a marked decrease in the frequency and severity of dyskinetic movements in response to acamprosate or memantine treatment. Relief of symptoms began within 48 hours of administration of treatment, and, if a patient discontinued treatment, symptoms returned immediately. This evidence supports my novel hypothesis that pharmacological agents such as acamprosate, memantine and dextromethorphan, or derivatives with similar pharmacodynamic actions, will be helpful in the treatment tardive movement disorders, including TD, tardive dystonia, tics disorders (including Tourette's), and focal dystonias. In particular, Case Reports 1 and 6 demonstrate that memantine can relieve blepharospasm and Meige syndrome associated with more extensive tardive movement disorders. By extension, I predict that memantine (with and without magnesium) and related agents will also be successful treatments for *idiopathic* blepharospasm and Meige syndrome, as well as other focal dystonias such as spasmodic torticollis, writer's cramp and other occupational dystonias.

Moreover, I have presented evidence elsewhere (See co-pending application Serial No. 09/193,892, filed November 18, 1998, entitled "Methods of Treating Tardive Dyskinesia and Other Movement Disorders") that magnesium, an antagonist of NMDA glutamate neurotransmission via ion channel blockade, can augment the therapeutic action of other NMDA receptor antagonists on movement disorders including tics, tardive dyskinesia and tardive dystonia. One can infer from this work, and it is herein demonstrated, that magnesium will augment the therapeutic effect of acamprosate, memantine and dextromethorphan for tics and tardive movement disorders, as well as for spontaneous movement disorders that closely mimic tardive movement disorders, including without limitation blepharospasm and Meige syndrome, as well as other focal dystonias such as spasmodic torticollis, writer's cramp and other occupational dystonias.

I tested whether administration of elemental magnesium would enhance the efficacy of acamprosate in the treatment of simple tics. In Case 5, it is demonstrated that supplementing memantine or acamprosate with magnesium salts alleviates tics better than memantine or acamprosate alone. Therefore, magnesium may be combined with any other agents that increase GABA transmission and/or decrease NMDA-glutamate transmission to further suppress simple tics.

Both calcium acetylhomotaurinate and magnesium salts or chelates are safe medications when given in appropriate dosage. Because magnesium acetylhomotaurinate yields the same magnesium ions and homotaurinate ions when it dissociates in the GI tract as does the mixture of acamprosate and magnesium salts. I infer that magnesium acetylhomotaurinate will also be a safe medication. Therefore, magnesium N-acetylhomotaurinate would be a safe and effective drug, with potentially greater efficacy for movement disorders than acamprosate (calcium), because of the NMDA-receptor blocking action of the magnesium ion. However, as noted earlier, it does not have the ideal molar ratio of magnesium to N-acetylhomotaurinate for maximal therapeutic effect. Therefore, a magnesium salt or chelate combined with a salt of N-acetylhomotaurine or a derivative is likely to be more efficacious as a treatment for movement disorder. Magnesium ion combination with acamprosate and related compounds is likely to alleviate symptoms of various hyperkinetic, dyskinetic, and dystonic movement disorders, for example multiple tics, Tourette syndrome, tardive dyskinesia, and blepharospasm, and other focal dystonias.

After beginning treatment with acamprosate, those patients who previously exhibited cognitive disorders showed functionally significant improvement in cognitive function (See Cases 1, 2, and 4). This evidence supports my novel hypothesis that acamprosate, or a derivative with similar pharmacodynamic actions, will be helpful in the treatment of hyperkinetic movement disorders, including dyskinesias and dystonias, and the cognitive impairment associated with them. Acamprosate and similar drugs have simultaneous actions on GABA neurotransmission and NMDA-type glutamate neurotransmission that may be synergistic in regards to the therapy of hyperkinetic, dyskinetic and dystonic movement disorders. To the extent that other related compounds and mixtures of compounds have similar simultaneous effects upon GABA and glutamate neurotransmission, these related compounds may have the same or similar action on movement disorders and their associated cognitive impairments. Related compounds include, but are not limited to other salts of N-acetylhomotaurinate (e.g., magnesium N-acetylhomotaurinate), acetylhomotaurinate base, homotaurine, derivatives of these compounds, and pro-drugs metabolized in the liver, blood, or brain to yield acetylhomotaurinate or analogues with similar pharmacodynamic effects on GABA and NMDA-type glutamate neurotransmission. Additionally, any derivatives or pro-drugs that are easily absorbed after oral administration, or have a long half-life are particularly desirable.

Acamprosate also has benefits for treating hyperkinetic or dyskinetic movement disorders other than TD. Case 3 shows it is useful in alleviating the peak-dose dyskinesia of Parkinson's disease treated with levodopa. Case 1 shows acamprosate can be used successfully to treat blepharospasm (a focal dystonia) and Meige syndrome when these are associated with TD, and suggests that it can be used successfully to treat idiopathic blepharospasm and Meige syndrome. Case 4 suggests that acamprosate is efficacious in treating simple tics, and, by extension, multiple tics and Gilles de la Tourette syndrome. By extension, acamprosate will likely benefit patients with movement disorders not induced by neuroleptics, that show clinical symptomatology identical with those of a neuroleptic-induced (tardive) movement disorder. In particular, as mentioned above, it may be efficacious in treating any of the focal dystonias, and in treating the involuntary movements of Huntington's disease. Patients with Huntington's disease also have a deficiency of GAD in the striatum, and are thought to suffer from neuronal death due to NMDA-receptor mediated excitotoxicity (DE Riley and AE Lang: Movement Disorders, in WG Bradley et al., editors, Neurology in Clinical Practice, Boston: Butterworth-Heinemann, 1991, p. 1568). These features of the disorder favor a positive response to acamprosate, a drug with joint actions on GABA and NMDA-receptors. Therefore, one can predict that patients will be relieved, at least in part, by acamprosate, memantine and dextronethorphan, alone or in combination with magnesium.

As mentioned above, one aspect of the method features improvements in the cognitive disorder associated with TD. The improvement in cognition and everyday functional performance seen during the treatment of TD, makes acamprosate particularly attractive for patients with the cognitive impairment that frequently accompanies TD.

The relationship between tardive dyskinesia and cognitive impairment is not fully understood. It is known that pre-existing cognitive impairment increases the risk that TD will develop in the event a patient receives neuroleptics over a long-term period. It is also known that treated schizophrenics with TD are more likely to show progressive cognitive deterioration that those without TD. However, it is not known whether treatment of TD will ameliorate the cognitive deficits associated with TD. Cases 1 , 2, and 4 discussed above, suggest that at least some treatments of TD can ameliorate such cognitive deficits. The prior art does not report that the administration of acamprosate, when used as a treatment for alcoholism, improved the patients' cognition, I infer that the improvement in cognition seen in Cases 1, 2, and 4 was related to the improvement in their movement disorders. This is consistent with the well-established involvement of the basal ganglia in cognitive processes (Sano et al., Basal Ganglia Diseases, in Fogel et al., (eds.), Neuropsychiatry, Williams and Wilkins, 1996)

Moreover, the fact that acamprosate is also known as an agent used in the treatment of alcoholism makes acamprosate particularly suited for the treatment of patients who have a history of alcoholism in addition to a hyperkinetic movement disorder. Once such group is patients with schizophrenia and alcoholism (so called "dual diagnosis" patients), who have TD, for which alcoholism is a risk factor.

## Claims

1. An agent suitable for oral administration for use in treating a hyperkinetic movement disorder wherein the agent is a GABA-receptor agonist and decreases the response of NMDA-type glutamate receptors; and wherein said agent is selected from the group consisting of: salts of N-acetylhomotaurinate, acetylhomotaurine base and derivatives thereof.

2. The agent according to claim 1, wherein said salt of N-acetylhomotaurinate is selected from the group consisting of: acamprosate (calcium N-acetylhomotaurinate), magnesium N-acetylhomotaurinate and lithium N-acetylhomotaurinate.

3. The agent according to claim 1, wherein said kinetic movement disorder is related to a deficiency in GABA in the basal ganglia or to glutamate-based excitotoxicity, or is selected from the group consisting of neuroleptic-induced movement disorder, focal dystonia, and a tic disorder.

4. The agent of claim 3, wherein:
(a) said neuroleptic-induced movement disorder is selected from the group consisting of tardive dyskinesia, tardive dystonia, tardive blepharospasm, and tardive akathisia; or
(b) said focal dystonia is selected from the group consisting of blepharospasm, Meige syndrome, spasmodic torticollis, spasmodic dysphonia, and writer's cramp; or
(c) said tic disorder is selected from the group consisting of simple tic, multiple tics, and Tourette's syndrome; or
(d) said focal dystonia is idiopathic blepharospasm.

5. The agent of any one of claims 1 to 3, wherein said hyperkinesia or said movement disorder is:
(a) tardive dyskinesia or involuntary movements similar to those seen in said tardive dyskinesia, or
(b) a peak-dose dyskinesia associated with Parkinson's disease or comprises involuntary movements similar to those seen in said peak-dose dyskinesia, or
(c) a movement disorder associated with Huntingtons's disease, or
(d) a movement disorder related to a deficiency in GABA in the basal ganglia, or
(e) a movement disorder related to NMDA-type glutamate neurotransmission, or
(f) a movement disorder that comprises involuntary movements similar to those seen in Tourette's syndrome, focal dystonias, blepharospasm and tics.

6. The agent of any one of the preceding claims, wherein said agent is metabolizable in the body to release acetylhomotaurinate ion into the body, and wherein the agent is selected from the group consisting of a derivative of acetylhomotaurinate, an ester of acetylhomotaurinate, or an ester of a derivative of acetylhomotaurine.

7. The agent of claim 6, wherein the agent is metabolized in the liver, blood or brain.

8. The agent of any one of the preceding claims, wherein said agent can ameliorate said movement disorder at non-toxic dosages.

9. The agent of any one of the preceding claims which is effective to improve cognitive symptoms in humans exhibiting said hyperkinetic movement disorder when said symptoms are determined by subjective performance, by a history of clear-cut changes in performance of everyday activities highly dependent on cognitive processes, by bedside mental status examination, or through the use of standard neuropsychological testing.

10. The agent of any one of the preceding claims, wherein said agent is acamprosate.

## Patentansprüche

1. Ein Agens, das für eine orale Verabreichung zur Verwendung in der Behandlung einer hyperkinetischen Bewegungsstörung geeignet ist, wobei das Agens ein GABA-Rezeptoragonist ist und die Antwort von NMDA-Typ Glutamatrezeptoren abschwächt; und wobei besagtes Agens aus der Gruppe ausgewählt ist, bestehend aus: Salzen von N-Acetylhomotaurinat, Acetylhomotaurin-Base und Derivaten davon.

2. Das Agens gemäß Anspruch 1, wobei besagtes Salz von N-Acetylhomotaurinat aus der Gruppe ausgewählt ist, bestehend aus: Acamprosat (Calcium-N-acetylhomotaurinat), Magnesium-N-acetylhomotaurinat und Lithium-N-acetylhomotaurinat.

3. Das Agens gemäß Anspruch 1, wobei besagte kinetische Bewegungsstörung mit einem Mangel an GABA in den Basalganglien oder mit einer Glutamat-basierten Erregungstoxizität verbunden ist oder aus der Gruppe ausgewählt ist, bestehend aus neuroleptisch-induzierter Bewegungsstörung, fokaler Dystonie oder einer Tic-Störung.

4. Das Agens nach Anspruch 3, wobei:
(a) besagte neuroleptisch-induzierte Bewegungsstörung aus der Gruppe ausgewählt ist, bestehend aus tardiver Dyskinesie, tardiver Dystonie, tardivem Blepharospasmus und tardiver Akathisie; oder
(b) besagte fokale Dystonie aus der Gruppe ausgewählt ist, bestehend aus Blepharospasmus, Meige Syndrom, spasmodischem Torticollis, spasmodischer Dysphonie und Schreibkrampf; oder
(c) besagte Tic-Störung aus der Gruppe ausgewählt ist, bestehend aus einfachem Tic, multiplen Tics und Tourette Syndrom; oder
(d) besagte fokale Dystonie idiopathischer Blepharospasmus ist.

5. Das Agens nach einem der Ansprüche 1 bis 3, wobei besagte Hyperkinesie oder besagte Bewegungsstörung ist:
(a) tardive Dyskinesie oder unwillkürliche Bewegungen, die denjenigen ähneln, die bei besagter tardiver Dyskinesie zu sehen sind, oder
(b) eine Peak-Dosis-Dyskinesie, die mit Parkinson Krankheit assoziiert ist oder unwillkürliche Bewegungen umfasst, die denjenigen ähneln, die bei besagter Peak-Dosis-Dyskinesie zu sehen sind, oder
(c) eine Bewegungsstörung, die mit Huntington Krankheit assoziiert ist, oder
(d) eine Bewegungsstörung, die mit einem Mangel an GABA in den Basalganglien verbunden ist, oder
(e) eine Bewegungsstörung, die mit einer NMDA-Typ Glutamatneurotransmission verbunden ist, oder
(f) eine Bewegungsstörung, die unwillkürliche Bewegungen umfasst, die denjenigen ähneln, die bei Tourette Syndrom, fokalen Dystonien, Blepharospasmus und Tics zu sehen sind.

6. Das Agens nach einem der vorhergehenden Ansprüche, wobei besagtes Agens im Körper metabolisierbar ist, um das Acetylhomotaurinat-Ion im Körper freizusetzen, und wobei das Agens aus der Gruppe ausgewählt ist, bestehend aus einem Derivat von Acetylhomotaurinat, einem Ester von Acetylhomotaurinat oder einem Ester eines Derivats von Acetylhomotaurin.

7. Das Agens nach Anspruch 6, wobei das Agens in der Leber, im Blut oder Gehirn metabolisiert wird.

8. Das Agens nach einem der vorhergehenden Ansprüche, wobei besagtes Agens besagte Bewegungsstörung bei nichttoxischen Dosierungen verbessern kann.

9. Das Agens nach einem der vorhergehenden Ansprüche, das wirksam ist, kognitive Symptome in Menschen zu verbessern, die besagte hyperkinetische Bewegungsstörung zeigen, wenn besagte Symptome durch subjektive Leistungsfähigkeit, durch eine Anamnese klar umrissener Veränderungen bei Verrichtung alltäglicher Aktivitäten, die von kognitiven Prozessen hoch abhängig sind, durch bettseitige Untersuchung des Mentalstatus oder durch die Anwendung neuropsychologischer Standardtests ermittelt werden.

10. Das Agens nach einem der vorhergehenden Ansprüche, wobei besagtes Agens Acamprosat ist.

## Revendications

1. Agent approprié pour l'administration orale destiné à être utilisé dans le traitement d'un trouble des mouvements hypercinétiques où l'agent est un agoniste de récepteur de GABA et diminue la réponse des récepteurs de glutamate de type NMDA ; et où ledit agent est choisi dans le groupe consistant en : les sels de N-acétylhomotaurinate, l'acétylhomotaurine base et leurs dérivés.

2. Agent selon la revendication 1 où ledit sel de N-acétylhomotaurinate est choisi dans le groupe consistant en : l'acamprosate (N-acétylhomotaurinate de calcium), le N-acétylhomotaurinate de magnésium et le N-acétylhomotaurinate de lithium.

3. Agent selon la revendication 1 où ledit trouble des mouvements cinétiques est lié à une déficience en GABA dans les ganglions basaux ou à une excitotoxicité basée sur le glutamate, ou est choisi dans le groupe consistant en le trouble des mouvements induit par des neuroleptiques, la dystonie focale et un trouble de type tic.

4. Agent selon la revendication 3 où :
(a) ledit trouble des mouvements induit par des neuroleptiques est choisi dans le groupe consistant en la dyscinésie tardive, la dystonie tardive, le blépharospasme tardif et l'acathisie tardive ; ou
(b) ladite dystonie focale est choisie dans le groupe consistant en le blépharospasme, le syndrome de Meige, le torticolis spasmodique, la dysphonie spasmodique et la crampe des écrivains ; ou
(c) ledit trouble de type tic est choisi dans le groupe consistant en les tics simples, les tics multiples et le syndrome de Tourette ; ou
(d) ladite dystonie focale est le blépharospasme idiopathique.

5. Agent selon l'une quelconque des revendications 1 à 3 où ladite hypercinésie ou ledit trouble des mouvements est :
(a) la dyscinésie tardive ou des mouvements involontaires similaires à ceux observés dans ladite dyscinésie tardive, ou
(b) une dyscinésie à dose maximale associée à la maladie de Parkinson ou comprend des mouvements involontaires similaires à ceux observés dans ladite dyscinésie à dose maximale, ou
(c) un trouble des mouvements associé à la maladie de Huntington, ou
(d) un trouble des mouvements associé à une déficience en GABA dans les ganglions basaux,
ou
(e) un trouble des mouvements lié à la neurotransmission au glutamate de type NMDA,
ou
(f) un trouble des mouvements qui comprend des mouvements involontaires similaires à ceux observés dans le syndrome de Tourette, les dystonies focales, le blépharospasme et les tics.

6. Agent selon l'une quelconque des revendications précédentes où ledit agent est métabolisable dans l'organisme pour libérer des ions acétylhomotaurinate dans l'organisme, et où l'agent est choisi dans le groupe consistant en un dérivé d'acétylhomotaurinate, un ester d'acétylhomotaurinate ou un ester d'un dérivé d'acétylhomotaurine.

7. Agent selon la revendication 6 où l'agent est métabolisé dans le foie, le sang ou le cerveau.

8. Agent selon l'une quelconque des revendications précédentes où ledit agent peut améliorer ledit trouble des mouvements à des dosages non toxiques.

9. Agent selon l'une quelconque des revendications précédentes qui est efficace pour améliorer les symptômes cognitifs chez les humains présentant ledit trouble des mouvements hypercinétiques quand lesdits symptômes sont déterminés par des performances subjectives, par une histoire de changements nets dans les performances des activités quotidiennes hautement dépendantes de processus cognitifs, par un examen de l'état mental au chevet ou par l'utilisation de tests neuropsychologiques standard.

10. Agent selon l'une quelconque des revendications précédentes où ledit agent est l'acamprosate.
